# EUROPEAN PATENT SPECIFICATION

(11) **EP 3 262 185 B1**
(45) Date of publication and mention of the grant of the patent: **26.08.2020**
(21) Application number: 15882959.8
(22) Date of filing: 27.02.2015
(51) Int. Cl.: C12Q 1/68, C12N 15/10, C12N 9/00

(54) **DNA DISPLAY AND METHODS THEREOF**
DNA-ANZEIGE UND VERFAHREN DAFÜR
AFFICHAGE D'ADN ET PROCÉDÉS ASSOCIÉS

(43) Date of publication of application: 03.01.2018
(73) Proprietor: The University of Hong Kong, Hong Kong (CN)
(72) Inventor: TANNER, Julian Alexander, Hong Kong (CN); KINGHORN, Andrew, Hong Kong (CN)
(74) Representative: HGF
(86) International application number: PCT/CN2015/073350
(87) International publication number: WO 2016/134521

(56) References cited:
- CN-A- 1 249 784
- DAVID R HALPIN ET AL: "DNA display III. Solid-phase organic synthesis on unprotected DNA", PLOS BIO, PUBLIC LIBRARY OF SCIENCE, UNITED STATES, vol. 2, no. 7, 1 January 2004 (2004-01-01) , pages 1031-1038, XP008141591, ISSN: 1544-9173, DOI: 10.1371/JOURNAL.PBIO.0020175
- JAESCHKE ET AL: "Oligonucleotide poly(ethylene glycol) conjugates: Synthesis, properties, and applications", WATER-SOLUBLE POLYMERS: SYNTHESIS, SOLUTION PROPERTIES AND APPLICATIONS, AMERICAN CHEMICAL SOCIETY, WASHINGTON, DC, US, [Online] vol. 680, 5 August 1997 (1997-08-05), pages 265-283, XP008095703, DOI: 10.1021/BK-1997-0680.CH018 ISBN: 978-0-541-23408-9 Retrieved from the Internet: URL:https://pubs.acs.org/doi/pdf/10.1021/b k-1997-0680.ch018>
- LEWIS FRASER ET AL: "Oligonucleotide Functionalised Microbeads: Indispensable Tools for High-Throughput Aptamer Selection", MOLECULES ONLINE, vol. 20, no. 12, 1 December 2015 (2015-12-01), pages 21298-21312, XP055482637, DE ISSN: 1433-1373, DOI: 10.3390/molecules201219766
- Kinghorn, Andrew Brian: "In silico and in vitro approaches to aptamer enrichment,selection and evolution", , 1 January 2016 (2016-01-01), XP002781781, Retrieved from the Internet: URL:http://hdl.handle.net/10722/240542 [retrieved on 2018-06-08]
- SCHLINGMAN, DANIEL J. ET AL.: 'A new method for the covalent attachment of DNA to a surface for single-molecule studies.' COLLOIDS SURF B BIOINTERFACES. vol. 83, no. 1, 31 March 2011, pages 91 - 95, XP027562737
- WANG, JIN PENG ET AL.: 'Particle Display: A Quantitative Screening Method for Generating High-Affinity Aptamers.' ANGEW CHEM INT ED ENGL. vol. 53, 05 May 2014, pages 4796 - 4801, XP002760830
- NAIMUDDIN, MOHAMMED ET AL.: 'Role of messenger RNA-bibosome complex in complementary DNA display.' ANALYTICAL BIOCHEMISTRY vol. 438, 01 April 2013, pages 97 - 103, XP028543456
- LU MING FENG ET AL.: 'Advance in study on in vitro display technology' CHINESE BULLETIN OF LIFE SCIENCES vol. 22, no. 8, 31 August 2010, pages 823 - 830, XP009504510

## Description

### 1. INTRODUCTION

The present disclosure generally relates to DNA display, methods of making a DNA display library, methods of using DNA display or a DNA display library for the selection of ligands that binds to a target. Described herein is a method of making a DNA display library for displaying RNA that is transcribed from a DNA template. The method comprises a process of ligation of a nucleoside triphosphate (RNA) to an oligonucleotide (DNA) via a bridging linker molecule. Also described herein is a method of capturing a transcriptional product (RNA) and linking it to its encoding template (DNA). Also described herein is a method of linking a transcriptional product (RNA) to a scaffold such as a solid support or bead via its encoding template (DNA). Described herein is a method of linking a translational product, such as but not limited to, a protein, peptide, antibody or enzyme, via its encoding transcriptional product (RNA) to a scaffold such as a solid support or a bead via its encoding template (DNA). More specifically described, the selection is an *in vitro* selection of binding ligands such as RNA aptamers and proteins. Also described herein is a kit that comprises the DNA display. Also disclosed herein is a double stranded DNA-RNA fusion comprising a DNA display template strand which is complementary to a DNA display coding strand except where said DNA display coding strand further comprises a linker molecule and a RNA molecule at its 5' end and wherein said RNA molecule is transcribed from the DNA display coding strand. Disclosed herein is a composition comprising the double stranded DNA-RNA fusion, as well as methods of making thereof, and use thereof in selecting binding ligands for a target.

### 2. BACKGROUND OF THE INVENTION

There are several display platforms that seek to achieve the objectives of displaying and isolating DNA and protein based on their function. Over the past 20 years *in vitro* selection of DNA and RNA aptamers using SELEX (US5696249 A) has yielded high affinity ligands. This type of in vitro selection had some distinct advantages over the *in vivo* selection methods used, mainly the large library sizes (>10¹⁵ molecules) capable of being screened (Osborne and Ellington 1997).

One advantage of the *in vivo* selection techniques such as yeast display (US6699658 B1) is the capability of selection using fluorescence-activated cell sorting (FACS). This significantly enhanced the scope of selection properties and accuracy of selection (Van Antwerp and Wittrup 2000). Yeast display has been an extremely successful strategy for the in vitro selection of antibodies largely due to FACS sorting. The disadvantages to yeast display and other cell surface display techniques (US5348867 A) is that the candidate library size is limited to the transfection efficiency of the organism used, the library size limit for yeast display is around 10¹⁰ molecules (Benatuil Perez et al. 2010).

Recently the technique particle display (Wang et al., 2014) was developed which utilises emulsion PCR to display an entire DNA aptamer library on beads, many copies of one aptamer sequence per bead. This has allowed for fluorescence-activated cell sorting (FACS) of DNA aptamers yielding binding affinities 1000 times stronger than previous DNA aptamer selection methods (Wang et al., 2014).

The molecular display techniques which are currently available and used in the field are mRNA display (Liu, Roberts et al. 2000) (US6261804 B1), ribosome display (He and Taussig 1998) (US6620587 B1), phage display (Scott and Smith 1990) (Sidhu, Weiss et al. 2012) (US8685893 B2), yeast display (Wittrup, Kranz et al. 2004) (US6699658 B1), bacterial display (Earhart, Francisco et al. 1993) (US5348867 A) and CIS display (Coomber, Eldridge et al. 2004) (US8679781 B2).

mRNA display (US6261804 B1), which is a protein displaying technique, utilizes puromycin, a mimic of both tyrosyl-tRNA and adenosine, attached to the end of an mRNA sequence via a linker and during translation the puromycin is inserted into the forming protein yielding a covalent link between the mRNA and the protein which the mRNA encodes.

Ribosome display (US6620587 B1), which is a protein displaying technique, involves the stalling of the ribosome at the end of translating mRNA to protein. This is due to the removal of the mRNA stop codon and the addition of a high magnesium concentration buffer which restricts ribosomal movement along the mRNA.

Phage display (US8685893 B2), which is a protein displaying technique, uses a bacteriophage genetically engineered such that its coat protein gene includes library sequences. This means the phage displays the protein outside while containing the DNA and RNA genetic information inside.

Yeast display (US6699658 B1), which is a protein displaying technique, involves displaying the protein of interest as a fusion protein with Aga2p on the surface of a yeast cell. The genetic information both RNA and DNA are contained within the yeast cell.

Bacterial display (US5348867 A), which is a protein displaying technique, involves the expression of the protein of interest as a fusion protein with a naturally surface displaying protein.

CIS display (US8679781 B2), which is a protein displaying technique, utilizes the protein RepA, a DNA replication initiator protein which binds to the DNA template from which it is expressed (McGregor et al., 2003). Using RepA, CIS display ligates a protein to the DNA from which it comes. CIS display has a few disadvantages. The RepA protein required is 351 amino acid residues in length (1053 bp of DNA sequence) and needs to be within a fusion protein with the library. This is a significant size so there is increased steric hindrance. Additionally the link between DNA and protein used in CIS display is non-covalent and therefore vulnerable to separation. Any capture based selection using CIS display would have an upper limit on the binding affinity which is the strength of the RepA/DNA interaction. This is the major shortcoming with CIS display.

Particle display, which is a DNA displaying technique, involves the conjugation of a primer to magnetic beads. Emulsion PCR can then be used to amplify library DNA onto the beads in such a way that each bead displays around 10⁵ copies of a single DNA aptamer sequence (Wang, Gong et al. 2014).

Previously demonstrated display techniques typically display proteins. No technique for displaying RNA exists. There is a need for a new platform which allows for RNA to be displayed on DNA

### 3. SUMMARY OF THE INVENTION

The present invention provides a method of producing an NTP-PEG-DNA conjugate as set forth in claims 1 to 4. The invention also provides a method of producing a DNA display library as set forth in any of claims 5 to 18. The invention further provides an NTP-PEG-DNA conjugate comprising a DNA polynucleotide and a nucleoside triphosphate (NTP), as set forth in claim 19, wherein the DNA polynucleotide and the NTP are conjugated via PEG.

Described herein is a rapid and efficient method of displaying RNA on DNA, and for the *in vitro* selection and *in vitro* evaluation to be applied to RNAs. The disclosure facilitates the isolation of RNA with desired properties from large pools of partially or completely random DNA sequences. Spatial informational loss is negated after completion of transcription by covalently attaching the RNA molecule to its encoding DNA. DNA display via the DNA display template can be used to capture RNA after transcription. Applications of this capture can be research-based, as in the quantification of transcription products, or more applied, as in correlating genotype (DNA) to phenotype (RNA) for in vitro evolution of RNA aptamers and proteins. Importantly DNA display connects the two other techniques particle display and mRNA display. Bridging this gap means that proteins, such as antibodies or enzymes, can be selected for on bead particles which is not possible without DNA display. This has significant application in protein directed evolution which produces antibodies and enzymes for cleaning products, food industry, biocatalyst alternate energy production, medical use, biochemistry and many other industries.

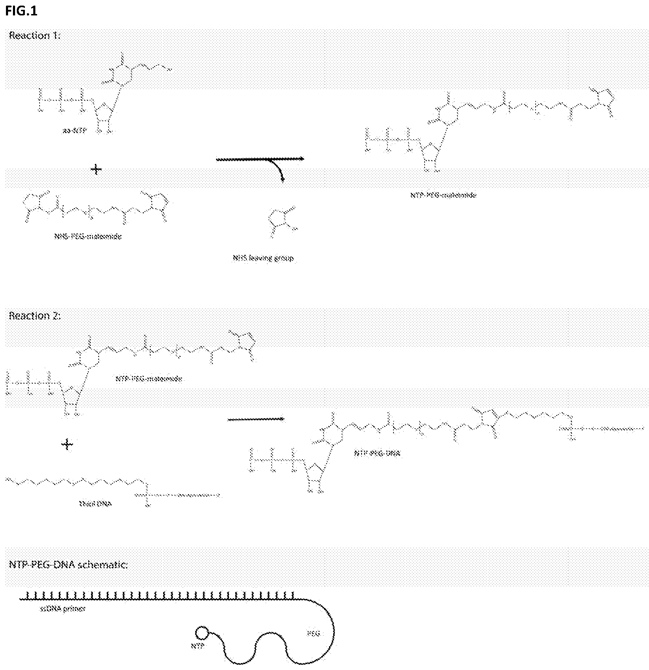

DNA display is a novel technique used to covalently link a newly transcribed RNA to its corresponding DNA template. This is achieved by using the DNA display template. The DNA display template is made by conjugating a nucleoside triphosphate (NTP) to a DNA template via a flexible PEG linker as denoted in figure 1. In general, the method consists of an in vitro or in situ extension/transcription protocol that generates RNA covalently linked to the 3' end of the DNA that encodes the RNA, i.e., a DNA-RNA fusion. The DNA display template linker molecule (DNA-Linker-NTP) may utilize PEG as a linker to form DNA display template PEG molecule (DNA-PEG-NTP). In this way, PEG is conjugated to the NTP via the reaction of an N-hydroxysuccinimide (NHS) functional group on the PEG to an aminoallyl group on the NTP. The NTP-PEG conjugate is then conjugated to the template DNA via the reaction of a maleimide functional group on the PEG to a thiol functional group on the template DNA. The final product is a DNA-PEG-NTP conjugate as seen in figure 1. This DNA-PEG-NTP conjugate can then be used for the capture of RNA during transcription.

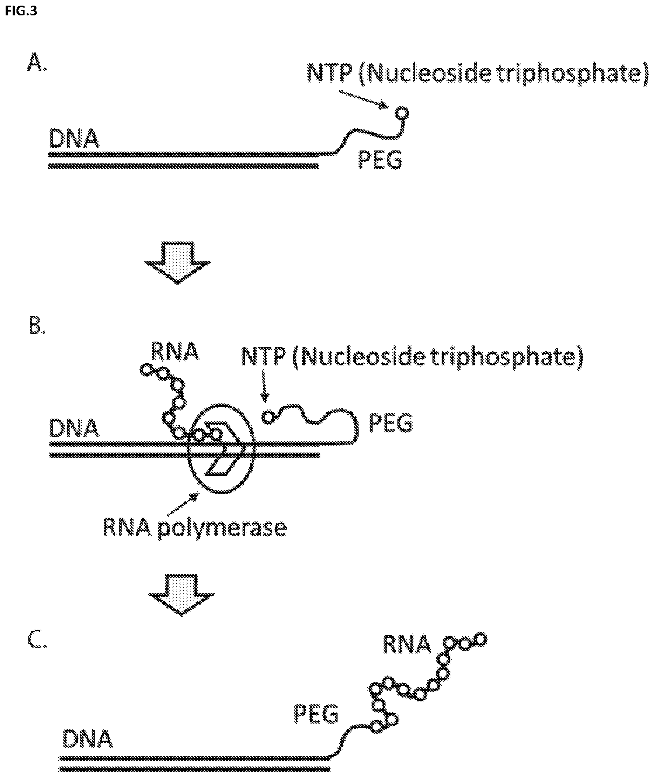

When the DNA display template is transcribed, the conjugated NTP is inserted into the newly formed RNA by RNA polymerase resulting in a covalent link between the template DNA and the RNA, which it encodes as seen in figure 3, which forms a DNA-RNA fusion.

This process of RNA capture can be repeated throughout selection rounds. The DNA from a selected DNA-RNA fusion can be amplified, NTP modified and transcribed to yield a progenic DNA-RNA fusion ready for the next round of selection. The ability to carry out multiple rounds of selection and amplification enables the enrichment and isolation of very rare molecules, e.g., one desired molecule out of a pool of 10¹⁵ members. This in turn allows the isolation of new or improved RNA or RNA aptamers which specifically recognize virtually any target or which catalyze desired chemical reactions.

Described herein is a method for selection of a desired RNA, involving the steps of: (a) providing a population of candidate DNA molecules, each of which includes a forward primer site, a T7 RNA polymerase promoter operably linked to a candidate RNA coding sequence where each is operably linked to a reverse primer site with a linker-NTP modification; (b) in vitro or in situ transcribing the candidate RNA coding sequences to produce a population of candidate DNA-RNA fusions; and (c) selecting a desired DNA-RNA fusion, thereby selecting the desired RNA.

Related and described herein is a method for selection of a DNA molecule which encodes a desired protein, involving the steps of: (a) providing a population of candidate DNA molecules, each of which includes a forward primer site, a T7RNA polymerase promoter operably linked to a candidate RNA coding sequence and each of which is operably linked to a reverse primer site with a linker-NTP modification; (b) in vitro or in situ transcribing the candidate RNA coding sequences to produce a population of DNA-RNA fusions; and (c) modifying the 5' end of the RNA with a puromycin linkage; and (d) in vitro or in situ translating the candidate RNA to produce a population of candidate DNA-RNA-protein fusions; and (e) selecting a desired protein, thereby selecting the desired DNA-RNA-protein fusion.

Also described herein is a method of making a DNA display library displaying RNA.

Also described herein is a method of producing a DNA display library comprising: (i) providing a population of DNA coding strands, each of which comprises a forward primer site, a T7RNA polymerase promoter, a DNA coding region and a reverse primer binding site; (ii) annealing each of the DNA coding strand to a forward primer; (iii) extending the primer in the presence of DNA polymerase to form a population of double stranded DNA display templates; (iv) denaturing the population of double stranded DNA display templates to form a population of single stranded DNA display template strand; (v) annealing a NTP-linker-DNA conjugate comprising a reverse primer to each of the single stranded DNA display template strand; and (vi) extending the reverse primer of the NTP-linker-DNA conjugate by PCR in the presence of DNA to form a population of double stranded DNA display template comprising a linker-NTP.

The NTP-linker-DNA conjugate may be produced by a method comprising the steps: (i) conjugating aminoallyl nucleoside triphosphate (aa-NTP) with a N-hydroxysuccinimide (NHS)-PEG-maleimide (NHS-PEG) forming NTP-PEG-maleimide (NTP-PEG); (ii) conjugating a DNA oligonucleotide functionalized with a reduced thiol (thiol-DNA) with the maleimide on the NTP-PEG forming NTP-PEG-DNA; and (iii) purifying the NTP-PEG-DNA conjugate.

The NTP-linker-DNA conjugate may comprise a linker that has a length of 200-300 angstrom. The linker may not comprise polynucleotides.

Described herein is a method of producing a DNA display template coated beads comprising: (i) providing a DNA display library coding strand comprising a forward primer site, a T7RNA polymerase promoter, a coding region and a reverse primer binding site; (ii) providing a forward primer conjugated bead; (iii) annealing the DNA display library coding strand to the forward primer conjugated bead; (iv) extending the primer on the forward primer conjugated bead by in the presence of DNA polymerase to form a bead comprising a double stranded DNA display template; (v) denaturing the double stranded DNA display template to form a bead comprising a single stranded DNA display template strand; (vi) annealing a NTP-PEG-DNA conjugate comprising a reverse primer with the single stranded DNA display template strand; (vii) extending the reverse primer of the NTP-PEG-DNA conjugate by PCR in the presence of DNA to form a bead comprising a double stranded DNA display template with PEG-NTP.

In one embodiment, the NTP-PEG-DNA conjugate is produced by the steps: (i) conjugating aminoallyl nucleoside triphosphate (aa-NTP) with a N-hydroxysuccinimide (NHS)-PEG-maleimide (NHS-PEG) forming NTP-PEG-maleimide (NTP-PEG); (ii) conjugating a DNA oligonucleotide functionalized with a reduced thiol (thiol-DNA) with the maleimide on the NTP-PEG forming NTP-PEG-DNA; and (iii) purifying the NTP-PEG-DNA conjugate.

The forward primer conjugated bead may be formed by an amino bond formed between carboxylic acid functionalized beads and an amine functionalized forward primer.

Described herein is a method of producing NTP-PEG-DNA conjugate comprising: (i) conjugating aminoallyl nucleoside triphosphate (aa-NTP) with a N-hydroxysuccinimide (NHS)-PEG-maleimide (NHS-PEG) forming NTP-PEG-maleimide (NTP-PEG); (ii) conjugating a DNA oligonucleotide functionalized with a reduced thiol (thiol-DNA) with the maleimide on the NTP-PEG forming NTP-PEG-DNA; and (iii) purifying the NTP-PEG-DNA conjugate.

The aa-NTP may be aminoallyl cytidine triphosphate

The NHS-PEG may be N-hydroxysuccinimide maleimide.

The reduced thiol may be a reduced 5' C6 S-S thiol modified DNA oligonucleotide.

The NTP-PEG-DNA conjugate may be a CTP-PEG-DNA conjugate.

The PCR may be emulsion PCR, where each bead displays multiple copies of a single coding strand sequence.

The emulsion may be broken and the double stranded DNA on the beads is denatured to single stranded DNA.

The DNA coding strand may be a reverse primer.

A method of preparing a library comprising RNA aptamers, said method comprising the steps of: (i) providing a population of DNA coding strands, each of which comprises a forward primer site, a T7RNA polymerase promoter, a coding region and a reverse primer binding site; (ii) annealing each of the DNA coding strand to a forward primer; (iii) extending the forward primer in the presence of DNA polymerase to form a population of double stranded DNA display templates; (iv) denaturing the population of double stranded DNA display templates to form a population of single stranded DNA display template strands; (v) annealing a NTP-PEG-DNA conjugate comprising a reverse primer to each of the single stranded DNA display template strand; and (vi) extending the reverse primer of the NTP-PEG-DNA conjugate by PCR in the presence of DNA to form a population of double stranded DNA display templates with PEG-NTP.

The NTP-PEG-DNA conjugate may be produced by the steps: (i) conjugating aminoallyl nucleoside triphosphate (aa-NTP) with a N-hydroxysuccinimide (NHS)-PEG-maleimide (NHS-PEG) forming NTP-PEG-maleimide (NTP-PEG); (ii) conjugating a DNA oligonucleotide functionalized with a reduced thiol (thiol-DNA) with the maleimide on the NTP-PEG forming NTP-PEG-DNA; (iii) purifying the NTP-PEG-DNA conjugate; and (iv) in vitro transcription of the DNA display template on beads comprising the double stranded DNA display template.

Also described herein is a method of preparing a library comprising RNA aptamer, said method comprising the steps of: (i) providing a population of DNA coding strands, each of which comprises a forward primer site, a T7RNA polymerase promoter, a coding region and a reverse primer binding site; (ii) providing forward primer conjugated beads; (iii) annealing each of the DNA display coding strand to the forward primer conjugated bead; (iv) extending the primer on the forward primer conjugated bead by in the presence of DNA polymerase to form a population of double stranded DNA display template; (v) denaturing the population of double stranded DNA display templates to form a population of single stranded DNA display template strands; (vi) annealing a NTP-PEG-DNA conjugate comprising a reverse primer with each of the single stranded DNA display template strand; (vii) extending the reverse primer of the NTP-PEG-DNA conjugate by PCR in the presence of DNA polymerase to form a population of double stranded DNA display template with PEG-NTP.

The NTP-PEG-DNA conjugate may be produced by the steps: (i) conjugating aminoallyl nucleoside triphosphate (aa-NTP) with a N-hydroxysuccinimide (NHS)-PEG-maleimide (NHS-PEG) forming NTP-PEG-maleimide (NTP-PEG); (ii) conjugating a DNA oligonucleotide functionalized with a reduced thiol (thiol-DNA) with the maleimide on the NTP-PEG forming NTP-PEG-DNA; (iii) purifying the NTP-PEG-DNA conjugate; and (iv) in vitro transcription of the DNA display template on beads comprising the double stranded DNA display template.

The forward primer conjugated bead may be formed by an amino bond formed between carboxylic acid functionalized beads and an amine functionalized forward primer.

Described herein is a method of screening for RNA aptamers comprising: (i) incubating the beads comprising the RNA aptamers with a labeled target protein for an amount of time sufficient for binding of the RNA aptamers with the target protein; (ii) washing to remove the unbound RNA aptamers; (iii) selecting RNA aptamers that binds to the target protein.

The method may further comprise the steps of: (i) amplifying the DNA templates of the selected RNA aptamers using PCR; and (ii) repeating rounds of PCR amplification sufficient to sequence the library to identify the isolated aptamers.

The method may further comprise the steps of: (i) amplifying the DNA templates of the selected RNA aptamers using PCR; (ii) constructing DNA display template coated beads as described above and subject to the next selection round.

The labeled target protein may be a fluorescent labeled target protein.

The selection may be FACs selection.

Also described herein is a method of providing a RNA encoding DNA display library comprising: (i) conjugating aminoallyl nucleoside triphosphate (aa-NTP) with a N-hydroxysuccinimide (NHS)-PEG-maleimide (NHS-PEG) forming NTP-PEG-maleimide (NTP-PEG); (ii) providing a population of DNA polynucleotides functionalized with a reduced thiol (thiol-DNA); (iii) conjugating the DNA polynucleotides functionalized with a reduced thiol (thiol-DNA) with the maleimide on the NTP-PEG forming NTP-PEG-DNA; and (iv) purifying the NTP-PEG-DNA conjugates.

Also described herein is a method of providing a RNA encoding DNA display library comprising: (i) conjugating aminoallyl nucleoside triphosphate (aa-NTP) with a N-hydroxysuccinimide (NHS)-PEG-maleimide (NHS-PEG) forming NTP-PEG-maleimide (NTP-PEG); (ii) providing a DNA library comprising DNA polynucleotides functionalized with a reduced thiol (thiol-DNA) ; (iii) conjugating DNA polynucleotides functionalized with a reduced thiol (thiol-DNA) with the maleimide on the NTP-PEG forming NTP-PEG-DNA; and (iv) purifying the NTP-PEG-DNA conjugates.

Described herein is a method of preparing a protein library comprising: (i) providing a population of DNA display coding strands, each of which comprises a forward primer site, a T7RNA polymerase promoter, a translation start codon, a coding region and a reverse primer binding site; (ii) providing forward primer conjugated beads; (iii) annealing each of the DNA display coding strand to the forward primer conjugated bead; (iv) extending the primer on the forward primer conjugated bead by PCR in the presence of DNA polymerase to form a bead comprising a double stranded DNA display template; (v) denaturing the double stranded DNA display template to form a bead comprising a single stranded DNA display template strand; (vi) annealing a NTP-PEG-DNA conjugate comprising a reverse primer with the single stranded DNA display template strand; (vii) extending the reverse primer of the NTP-PEG-DNA conjugate by PCR in the presence of DNA polymerase to form a bead comprising a double stranded DNA display template with PEG-NTP; (viii) in vitro transcription of the DNA display template on beads comprising the double stranded DNA display template producing DNA-RNA fusion comprising a DNA portion and a RNA portion; (ix) in vitro translating the RNA portion to produce a population of RNA-protein fusions, thereby producing a protein library.

The RNA-protein fusion may comprise an mRNA linked to the protein via a puromycin linkage.

The forward primer conjugated beads may be formed by an amino bond formed between carboxylic acid functionalized beads and an amine functionalized forward primer.

Described herein is a method of selecting a target protein comprising: (i) providing a DNA coding strand comprising a forward primer site, a T7RNA polymerase promoter, a translation start codon, a random library region and a reverse primer binding site; (ii) providing a forward primer conjugated bead (an amino bond formed between carboxylic acid functionalized beads and an amine functionalized forward primer); (iii) annealing the DNA display library coding strand to the forward primer conjugated bead; (iv) extending the primer on the forward primer conjugated bead by PCR in the presence of DNA polymerase to form a bead comprising a double stranded DNA display template; (v) denaturing the double stranded DNA display template to form a bead comprising a single stranded DNA display template strand; (vi) annealing a NTP-PEG-DNA conjugate comprising a reverse primer with the single stranded DNA display template strand; (vii) extending the reverse primer of the NTP-PEG-DNA conjugate by PCR in the presence of DNA polymerase to form a bead comprising a double stranded DNA display template with PEG-NTP; (viii) in vitro transcription of the DNA display template on beads comprising the double stranded DNA display template producing DNA-RNA fusion comprising a DNA portion and a RNA portion; (ix) in vitro translating the RNA portion to produce a population of RNA-protein fusions, thereby producing a protein library; and (x) selecting a desired RNA-protein fusion based on fusion binding or activity, thereby selecting said desired protein and said nucleic acid encoding said protein.

The desired protein may be antibodies, fragments of antibodies, aptazymes and enzymes.

The desired protein may be selected by FACs.

The nucleotide triphosphate (aa-NTP) may be adenosine triphosphate (ATP), guanosine triphosphate (GTP), cytidine triphosphate (CTP), 5-methyluridine triphosphate (m5UTP), uridine triphosphate (UTP), any unnatural nucleoside triphosphate.

The RNA polymerase substrate may be any alternate linking chemistry added for attachment to the PEG.

The linker may be PEG;
for example PEG3100, PEG 3200, PEG3300, PEG3400, PEG3500 or PEG3600.

In some methods, the linker does not comprise polynucleotides.

In some methods, the linker has a length of 100-200 angstrom, 200-250 angstrom, 250-280 angstrom, 280-300 angstrom, 300-400 angstrom.

Described herein is a DNA-RNA fusion comprising an deoxyribopolynucleotide, linker molecule and a nucleoside triphosphate.

Described herein is a double stranded DNA-RNA fusion comprising a DNA display template strand which is complementary to a DNA display coding strand except where said DNA display coding strand further comprises a linker molecule and a RNA molecule at its 5' end and wherein said RNA molecule is transcribed from the DNA display coding strand.

The linker molecule may be polyethylene glycol.

Also described herein is a method of capturing a transcriptional product to a scaffold.

The scaffold may be a solid support.

The solid support may be a bead, a membrane or a filter.

The population of DNA coding strands, DNA coding sequence, candidate DNA sequence includes at least 10⁹, preferably, at least 10¹⁰, more preferably, at least 10¹¹, 10¹², or 10¹³, and, most preferably, at least 10¹⁴ different RNA; the in vitro transcription reaction is carried out using a buffered solution containing ATP, CTP, GTP, UTP, DTT, RNase inhibitor, T7 RNA polymerase and the DNA display template;
the selection step involves binding of the desired RNA to an immobilized binding partner; the selection step involves assaying for a functional activity of the desired RNA; the DNA molecule is amplified; the method further involves repeating the steps of the above selection methods; the method further involves transcribing an RNA molecule from the DNA molecule and repeating the cycle.

Also described herein is a method for selection of a desired RNA or desired DNA through enrichment of a sequence pool. This method involves the steps of: (a) providing a population of candidate DNA molecules, each of which comprises a forward primer site, a T7RNA polymerase promoter, a DNA coding region and a reverse primer binding site; (ii) annealing each of the DNA coding strand to a forward primer; (iii) extending the primer in the presence of DNA polymerase to form a population of double stranded DNA display templates; (iv) denaturing the population of double stranded DNA display templates to form a population of single stranded DNA display template strand; (v) annealing a NTP-linker-DNA conjugate comprising a reverse primer to each of the single stranded DNA display template strand; (vi) extending the reverse primer of the NTP-linker-DNA conjugate by PCR in the presence of DNA to form a population of double stranded DNA display template comprising a linker-NTP; (vii) in vitro or in situ transcribing the candidate DNA coding sequences to produce a population of candidate DNA-RNA fusions; (viii) contacting the population of DNA-RNA fusions with a binding partner specific for either the DNA portion or the RNA portion of the DNA-RNA fusion under conditions which substantially separate the binding partner-DNA-RNA fusion complexes from unbound members of the population; (ix) releasing the bound DNA-RNA fusions from the complexes; and (x) contacting the population of DNA-RNA fusions from step (ix) with a binding partner specific for the RNA portion of the desired DNA-RNA fusion under conditions which substantially separate the binding partner-DNA-RNA fusion complex from unbound members of said population, thereby selecting the desired RNA.

The method may further involve repeating steps (i) through (x). In addition, for these repeated steps, the same or different binding partners may be used, in any order, for selective enrichment of the desired DNA-RNA fusion.

Step (x) may involve the use of a binding partner specific for the RNA portion of the desired fusion. This step is carried out following reverse transcription of the RNA portion of the fusion to generate a DNA which encodes the desired protein. If desired, this DNA may be isolated and/or PCR amplified.

Also described herein are methods for producing libraries (for example, protein, DNA-RNA libraries, RNA aptamer libraries) or methods for selecting desired molecules (for example, protein, DNA, or RNA molecules or molecules having a particular function or altered function).

Also described herein are kits for carrying out any of the selection methods described herein.

Also described herein is a microchip that includes an array of immobilized single-stranded nucleic acids, the nucleic acids being hybridized to DNA-RNA fusions. Preferably, the RNA component of the DNA-RNA fusion is encoded by the DNA.

### 4. BRIEF DESCRIPTION OF THE DRAWINGS

**FIG. 1** **Schematic of chemical ligation reactions used to create the NTP-PEG-DNA conjugate.** In reaction 1 aminoallyl nucleoside triphosphate (aa-NTP) is conjugated to an N-hydroxysuccinimde (NHS) on a heterobifunctional PEG. This results in the conjugate along with an NHS leaving group which along with excess aa-NTP is removed using size exclusion chromatography prior to reaction 2. In reaction 2 DNA oligonucleotide functionalised with a reduced thiol is conjugated to the maleimide on the functional PEG-NTP conjugate from reaction 1. Following this reaction anion exchange chromatography is used to purify the final NTP-PEG-DNA product.

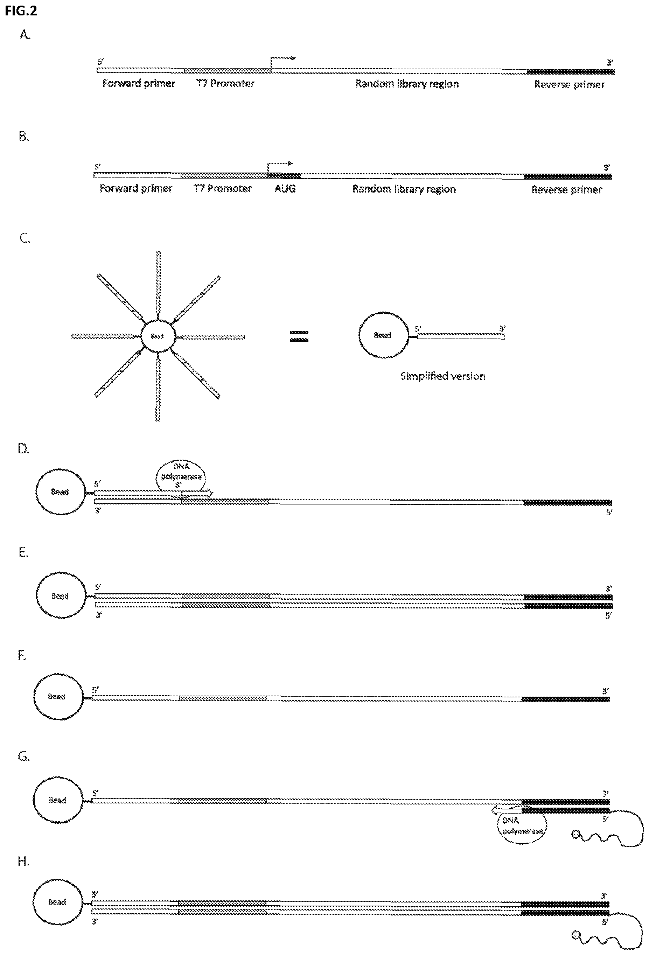

**FIG. 2** **Construction of DNA display template coated** beads. A) DNA display library coding strand design. The DNA display library coding strand includes a forward primer site, a T7 RNA polymerase promoter, a random library region and a reverse primer binding site. B) Protein encoding DNA display library coding strand design. The Protein encoding DNA display library coding strand includes a forward primer site, a T7 RNA polymerase promoter, an AUG start codon, a random library region and a reverse primer binding site. C) Forward primer conjugated beads. Forward primer to bead conjugation may be an amino bond formed between carboxylic acid functionalised beads and an amine functionalised forward primer. D) DNA display template strand extension by DNA polymerase. The DNA display coding strand is annealed to FP bead and iterative cycles of PCR allow for DNA polymerase to form beads decorated with double stranded DNA display template. E) The double stranded DNA display template coupled to bead which is formed following PCR. F) Bead decorated with single stranded DNA display template strand. To achieve a bead decorated with single stranded DNA display template strand the double stranded DNA display template coupled to bead is denatured. G) DNA display coding strand extension by DNA polymerase using NTP-PEG-DNA reverse primer. The DNA display template strand is annealed to NTP-PEG-DNA reverse primer and iterative cycles of PCR allow for DNA polymerase to form beads decorated with double stranded DNA display template with NTP attached via a PEG linker. H) Complete, bead coupled, double stranded DNA display template with NTP attached via a PEG linker.
**FIG. 3** **Schematic diagram of DNA display.** A) Complete, double stranded DNA display template with NTP attached via a PEG linker. B) Transcription of DNA display template with conjugated NTP being inserted into newly forming RNA chain. C) RNA displayed on DNA template. D) The DNA display template undergoing transcription to yield RNA displayed n DNA. Upon transcription of the DNA template, the conjugated nucleoside triphosphate is inserted into the newly formed RNA by RNA polymerase resulting in a covalent link between the template DNA and the RNA which it encodes. DNA display for displaying RNA via the DNA display template can be used to capture RNA after transcription.

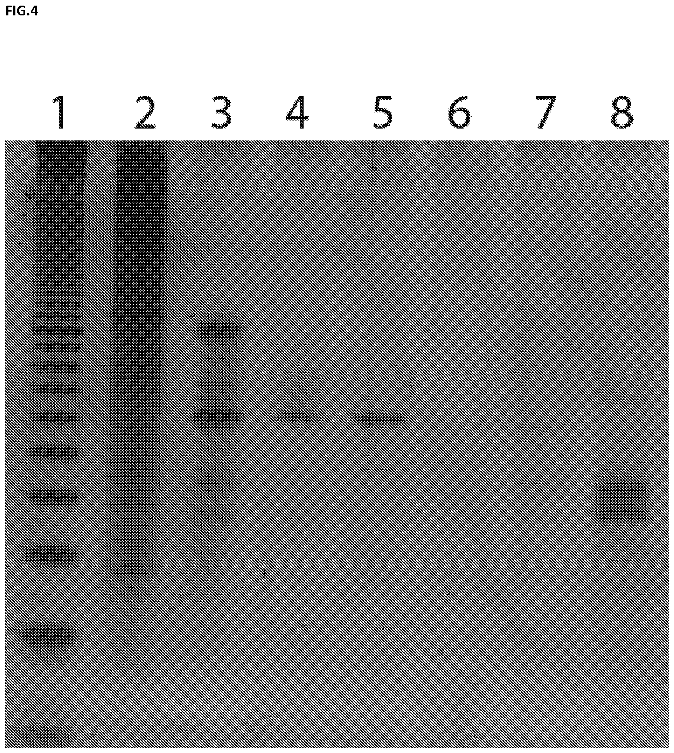

**FIG. 4** **Polyacrylamide gel electrophoresis demonstrating DNA display.** Lane 1 is 10bp DNA ladder. Lane 2 is RNA ladder. Lane 3 is shows DNA display template with transcription. Lane 4 shows DNA display template without transcription. Lane 5 shows DNA display template with transcription and RNAse treatment. Lane 6 shows the loss of DNA from the beads following DNAse treatment. Lane 7 shows the flow through after DNAse treatment. Lane 8 is the free RNA after transcription. The increase in mass due to attachment of transcribed RNA can be seen in the band shift when comparing lane 3, the DNA display template with transcription, and lane 4, the DNA display template without transcription. Lane 5, the DNA display template with transcription followed by RNAse treatment, confirms that this shift is due to RNA as when the RNAse degrades the attached RNA the band shifts back to the original DNA only position.

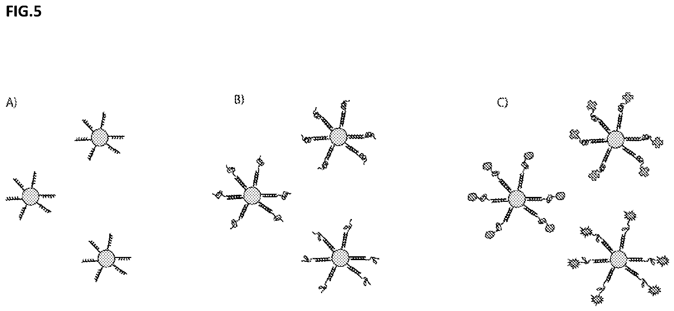

**FIG. 5** **Schematic diagram of various displays.** 1. Particle display 2. Particle display and DNA display 3. Particle display, DNA display and mRNA display.

### 4.1 DEFINITIONS

As used herein, by a "population" is meant more than one molecule (for example, more than one RNA, DNA, or DNA-RNA fusion molecule). Because the disclosed methods facilitate selections which begin, if desired, with large numbers of candidate molecules, a "population" preferably means more than 10⁹ molecules, more preferably, more than 10¹¹, 10¹², or 10¹³ molecules, and, most preferably, more than 10¹³ molecules.
By "selecting" is meant substantially partitioning a molecule from other molecules in a population. As used herein, a "selecting" step provides at least a 2-fold, preferably, a 30-fold, more preferably, a 100-fold, and, most preferably, a 1000-fold enrichment of a desired molecule relative to undesired molecules in a population following the selection step. As indicated herein, a selection step may be repeated any number of times, and different types of selection steps may be combined in a given approach.
By a "protein" is meant any two or more naturally occurring or modified amino acids joined by one or more peptide bonds. "Protein" and "peptide" are used interchangeably herein.
By "RNA" is meant a sequence of two or more covalently bonded, naturally occurring or modified ribonucleotides.
By a "start codon" is meant three bases which signal the beginning of a protein coding sequence. Generally, these bases are AUG (or ATG); however, any other base triplet capable of being utilized in this manner may be substituted.
By "covalently bonded" means either directly through a covalent bond or indirectly through another covalently bonded sequence.
By an "altered function" is meant any qualitative or quantitative change in the function of a molecule.
By "binding partner," as used herein, is meant any molecule which has a specific, covalent or non-covalent affinity for a portion of a desired DNA-RNA fusion. Examples of binding partners include, without limitation, members of antigen/antibody pairs, protein/inhibitor pairs, receptor/ligand pairs (for example cell surface receptor/ligand pairs, such as hormone receptor/peptide hormone pairs), enzyme/substrate pairs (for example, kinase/substrate pairs), lectin/carbohydrate pairs, oligomeric or heterooligomeric protein aggregates, DNA binding protein/DNA binding site pairs, RNA/protein pairs, and nucleic acid duplexes, heteroduplexes, or ligated strands, as well as any molecule which is capable of forming one or more covalent or non-covalent bonds (for example, disulfide bonds) with any portion of an DNA-RNA fusion.
By a "solid support" is meant, without limitation, any column (or column material), bead, test tube, microtiter dish, solid particle (for example, agarose or sepharose), microchip (for example, silicon, silicon-glass, or gold chip), or membrane (for example, the membrane of a liposome or vesicle) to which an affinity complex may be bound, either directly or indirectly (for example, through other binding partner intermediates such as other antibodies or Protein A), or in which an affinity complex may be embedded (for example, through a receptor or channel).

### 5. DETAILED DESCRIPTION OF THE INVENTION

Molecules that bind specifically to other molecules are essential for a plethora of biomedical and analytical applications, such as, therapeutics, diagnostics, laboratory research, and many facets of analytical sciences. The present disclosure provides a number of significant advantages. The present disclosure allows for repeated rounds of selection using populations of candidate molecules of considerable length. The present disclosure relates to a novel process of DNA display for the discovery and evolution of binding ligands for biomedical and analytical applications. These binding ligands includes, but are not limited to, nucleic acids or proteins (including, but are not limited to, antibodies, antibody fragments, peptides, polypeptides). Besides selecting for binding ligands, DNA display is used to select for and improve catalytic activities including, but are not limited to, enzymes, ribozymes and aptazymes.

The disclosed DNA display is a process by which a link is made between the binding ligand back to the DNA which encoded the molecule. Disclosed herein is the DNA display template is made up of three parts: the DNA template, the linker and the NTP, which is an adaptor for this process. The link allows a selection of a specific molecule that binds to a desired target in a mixed population of binding molecules. The DNA which encodes the binding molecule can then be used to produce the binding molecule or produce new binding molecules. The DNA-RNA fusion provides improved methods to select binding molecules that are useful in pharmaceuticals and biotechnology.

Another advantage is that the present selection and directed evolution technique can make use of very large and complex libraries of candidate sequences. Large library size provides an advantage for directed evolution applications. The candidate pool size has the potential to be in the order of 10¹⁵ candidates. Random regions may be screened in isolation or within the context of a desired DNA-RNA fusion. Most if not all possible sequences may be expressed in candidate pools of DNA-RNA fusions. Creating binding molecules for a target involves iterative cycles of selection. A mixed pool of binding molecules is exposed to the target, the weak binders are washed off and the stronger binders are kept. These stronger binders are then bred together or amplified and then make up the new pool of binding molecules for the next cycle of binding molecule evolution. This cycle is repeated, increasing the binding ability of the pool of molecules until very strong binding molecules are obtained. For aptamer on bead selection techniques there is spatial informational loss after following transcription. This informational loss can be avoided using the present DNA display disclosed herein.

Described herein is a general method for the selection of RNA from a population of RNAs with desired functions using fusions in which the RNAs are covalently linked to their encoding DNAs. These DNA-RNA fusions are synthesized by in vitro or in situ extension and transcription of DNA pools containing a forward primer region, a T7RNA polymerase promoter, a DNA coding region and a reverse primer region(FIG. 2A). The covalent link between the RNA and the DNA (in the form of an NTP-linker-DNA conjugate) allows the spatial information of the DNA to be intrinsically linked to that of the RNA which it encodes. This is critical for aptamer on bead selection techniques such as FACs.

### 5.1 Synthesis of NTP-LINKER-DNA

In order to generate templates for the DNA-RNA fusions, NTP-Linker-DNA conjugate is made. Aminoallyl nucleoside triphosphate (aa-NTP) is conjugated to a linker. The linker may be a heterobifunctional PEG. The aa-NTP is conjugated to an N-hydroxysuccinimide (NHS) on the heterobifunctional PEG. This results in the conjugate with an NHS leaving group where excess aa-NTP is removed using size exclusion chromatography (FIG. 1, Reaction 1). DNA oligonucleotide functionalized with a reduced thiol is conjugated to the maleimide on the functional PEG-NTP conjugate from reaction 1. Following this reaction, anion exchange chromatography is used to purify the final NTP-PEG-DNA conjugate.

Aminoallyl cytidine triphosphate (aa-CTP) is conjugated to a heterobifunctional NHS-PEG-maleimide linker via a NHS ester forming an amide bond as in figure 1. The CTP-PEG- maleimide conjugate is then reacted with a reduced 5' C6 S-S thiol modified DNA oligonucleotide to form CTP-PEG-DNA as in figure 1.
The DNA oligonucleotide may be the reverse primer from figure 2.

The nucleoside triphosphate may be adenosine triphosphate (ATP), guanosine triphosphate (GTP), cytidine triphosphate (CTP), 5-methyluridine triphosphate (m5UTP), uridine triphosphate (UTP), any unnatural nucleoside triphosphate or any RNA polymerase substrate with any alternate linking chemistry added for attachment to the PEG.

A PEG linker may be used with thiol-maleimide linking chemistry. Linkers other than PEG can be used with an array of linking chemistries.

The length of the PEG linker used is of importance as too short will not allow the conjugated NTP to insert into the newly forming RNA strand and too long may result in early insertion or possible increase the likelihood of nonspecific NTP insertion.

The NTP-linker-DNA conjugate may comprise a linker that has a length of 200-300 angstrom. The linker may have a length of 280 angstrom.

The linker may not comprise polynucleotides.

The PEG may be PEG3100, PEG 3200, PEG3300, PEG3400, PEG3500 or PEG3600; more particularly the PEG is PEG3400.

### 5.2 CONSTRUCTION OF DNA DISPLAY TEMPLATE COATED BEADS

Shown in FIG. 2 is a DNA display scheme of the present disclosure. The steps involved in the construction of the DNA display template coated beads are generally carried out as follows.
(A) DNA display coding strand design (FIG. 2A). The DNA display library coding strand design for a typical RNA encoding DNA display library is shown in FIG. 2A. The DNA display coding strand includes a forward primer site, a T7 RNA polymerase promoter, a random DNA coding region up to but not restricted to 30kb, and a reverse primer binding site. In other schemes, the DNA coding region is at least but not limited to 10-15kb, 15-20kb, 20-25kb, 25-30kb, 30-35kb, 35-40kb, 40-45kb, 45-50kb.
   In one embodiment, for selection using flow cytometry, the coding strand library is amplified onto beads.
(B) Forward primer conjugated beads (FIG. 2C). The forward primer is conjugated to the bead through an amino bond formed between carboxylic acid functionalized beads and an amine functionalized forward primer.
(C) DNA display template strand extension by DNA polymerase (FIG.2D). In order to amplify the DNA display coding strand, a DNA display template strand complementary to the DNA display coding strand (FIG. 2A) is extended by DNA polymerase. The DNA display coding strand is annealed to FP bead and amplified using emulsion PCR in such a way that each bead displays many copies of a single DNA display coding strand similarly to Wang et al. (Wang et al., 2014) (FIG. 2D).
(D) The double stranded DNA display template coupled to bead is formed following PCR (FIG. 2E).
(E) Bead decorated with single stranded DNA display template strand. After the emulsion is broken and the double stranded DNA (FIG. 2E) on the beads is denatured to single stranded DNA display template strand (FIG. 2F).
(F) DNA display coding strand is extended by DNA polymerase using NTP-PEG-DNA reverse primer. The NTP-PEG-DNA of which the DNA is a reverse primer is annealed to the single stranded DNA coding strand and amplified by PCR in the presence of DNA polymerase (FIG. 2G).
(G) Complete, bead coupled, double stranded DNA display template with NTP attached via a PEG linker (FIG. 2H).

Another DNA display is constructed as described above but does not include beads.

### 5.3 CONSTRUCTION OF DNA DISPLAY TEMPLATE

The steps involved in the construction of the DNA display template are generally carried out as follows.
(A) DNA display coding strand design. The DNA display library coding strand design for a typical RNA encoding DNA display library is shown in FIG. 2A. The DNA display coding strand includes a forward primer site, a T7 RNA polymerase promoter, a random DNA coding region and a reverse primer binding site.
   In one DNA display template, the DNA display is not associated with beads.
(B) DNA display template strand extension by DNA polymerase. In order to amplify the DNA display coding strand, a DNA display template strand complementary to the DNA display coding strand is extended by DNA polymerase. The DNA display coding strand is and amplified using.
(C) The double stranded DNA display template is formed following PCR.
(D) After the emulsion is broken and the double stranded DNA is denatured to single stranded DNA display template strand.
(E) DNA display coding strand is extended by DNA polymerase using NTP-PEG-DNA reverse primer. The NTP-PEG-DNA of which the DNA is a reverse primer is annealed to the single stranded DNA coding strand and amplified by PCR in the presence of DNA polymerase.
(F) DNA display coding strand is extended by DNA polymerase using NTP-PEG-DNA reverse primer. The NTP-PEG-DNA of which the DNA is a reverse primer is annealed to the single stranded DNA coding strand and amplified by PCR in the presence of DNA polymerase.
(G) Double stranded DNA display template with NTP attached via a PEG linker.

### 5.4 CONSTRUCTION OF DNA DISPLAY TEMPLATE COATED BEADS DISPLAYING PROTEIN CODING RNA

The steps involved in the construction of Protein Coding DNA display template coated beads are generally carried out as follows.
(A) Protein coding DNA display coding strand design (FIG. 2B). The DNA display library coding strand design for displaying a protein coding RNA in a DNA display library is shown in FIG. 2B. The protein encoding DNA display coding strand includes a forward primer site, a T7 RNA polymerase promoter, a start codon, a random DNA coding region and a reverse primer binding site.
   In one example, for selection using flow cytometry, the coding strand library is amplified onto beads.
(B) Forward primer conjugated beads. The forward primer is conjugated to the bead through an amino bond formed between carboxylic acid functionalized beads and an amine functionalized forward primer.
(C) DNA display template strand extension by DNA polymerase. In order to amplify the DNA display coding strand, a DNA display template strand complementary to the protein coding DNA display coding strand (FIG. 2B) is extended by DNA polymerase. The DNA display coding strand is annealed to FP bead and amplified using emulsion PCR in such a way that each bead displays many copies of a single DNA display coding strand similarly to Wang et al. (Wang et al., 2014) (FIG. 2D).
(D) The double stranded DNA display template coupled to bead is formed following PCR.
(E) Bead decorated with single stranded DNA display template strand. After the emulsion is broken and the double stranded DNA (FIG. 2E) on the beads is denatured to single stranded DNA display template strand.
(F) DNA display coding strand is extended by DNA polymerase using NTP-PEG-DNA reverse primer. The NTP-PEG-DNA of which the DNA is a reverse primer is annealed to the single stranded DNA coding strand and amplified by PCR in the presence of DNA polymerase.
(G) Complete, bead coupled, double stranded DNA display template with NTP attached via a PEG linker.

In another example, the DNA display is constructed as described above but does not include beads.

### 5.5 CONSTRUCTION OF DNA DISPLAY TEMPLATE DISPLAYING PROTEIN CODING RNA

The steps involved in the construction of the DNA display template are generally carried out as follows.
(A) Protein coding DNA display coding strand design (FIG. 2B). The DNA display library coding strand design for displaying a protein coding RNA in a DNA display library is shown in FIG. 2B. The protein encoding DNA display coding strand includes a forward primer site, a T7 RNA polymerase promoter, a start codon, a random DNA coding region and a reverse primer binding site.
(B) DNA display template strand extension by DNA polymerase. In order to amplify the DNA display coding strand, a DNA display template strand complementary to the DNA display coding strand is extended by DNA polymerase. The DNA display coding strand is and amplified using.
(C) The double stranded DNA display template is formed following PCR.
(D) After the emulsion is broken and the double stranded DNA is denatured to single stranded DNA display template strand.
(E) DNA display coding strand is extended by DNA polymerase using NTP-PEG-DNA reverse primer. The NTP-PEG-DNA of which the DNA is a reverse primer is annealed to the single stranded DNA coding strand and amplified by PCR in the presence of DNA polymerase.
(F) DNA display coding strand is extended by DNA polymerase using NTP-PEG-DNA reverse primer. The NTP-PEG-DNA of which the DNA is a reverse primer is annealed to the single stranded DNA coding strand and amplified by PCR in the presence of DNA polymerase.
(G) Double stranded DNA display template with NTP attached via a PEG linker.

### 5.7 CONSTRUCTION OF DNA DISPLAY TEMPLATE COATED BEADS

Instead of linking the NTP-PEG to a DNA oligonucleotide primer, the NTP-PEG can be conjugated directly to library DNA. This means less chain extension rounds are required per selection round but every round must then include a conjugation step.

In one example, the DNA display library coding strand design for a typical RNA encoding DNA display library includes a forward primer site, a T7 RNA polymerase promoter, a random DNA coding region directly linked to NTP-linker. In one example, the linker is PEG. In vitro transcription is then performed on the DNA display template and the PEG linker conjugated NTP incorporates into the forming RNA chain. This resulted in RNA displayed on DNA template. The DNA display can be associated with beads or without beads. In one example, the DNA coding template strand comprises a start codon. In one example, the DNA coding template strand does not comprise a start codon. In one example, the start codon is AUG.

### 5.8 DISPLAY OF RNA ON DNA DISPLAY TEMPLATE

In vitro transcription is then performed on the DNA display template coated beads and the PEG linker conjugated NTP incorporates into the forming RNA chain (FIG. 3). This resulted in RNA displayed on DNA template. Using this method, in one example, a library of RNA aptamers can be prepared which can then undergo selection.

### 5.9 SELECTION AND AMPLIFICATION

The RNA aptamers on beads can then be incubated with fluorescent target protein and washed to be ready for FACs selection. The selected RNA aptamers can then have their DNA templates amplified using particle PCR (Wang et al., 2014). If sufficient selection rounds have taken place this library can sequenced to identify the isolated aptamers, otherwise the library can be used for the construction of the next generation of DNA display template coated beads for the next selection round.

In the case of selection of proteins on beads, a protein encoding DNA display library (figure 2B) can be attached to the beads using emulsion PCR, as above. In vitro transcription can then be performed to yield a library of RNA on beads. The technique of mRNA display (US6261804 B1) (Wilson, Keefe et al. 2001) can then be performed on the RNA displaying beads to yield protein displaying beads. This is in effect display of proteins on RNA via the DNA display template which is attached to the beads. It is then possible to perform FACs selection of proteins on beads.

### 5.10 PREPARATION OF DNA TEMPLATE

As a step toward generating the DNA-RNA fusions, the DNA portion of the fusion is synthesized. This may be accomplished by direct chemical DNA synthesis or, more commonly, is accomplished by extension of DNA using PCR in the presence of DNA polymerase and purification of any double-stranded DNA template.

Such DNA templates may be created by any standard technique (including any technique of recombinant DNA technology, chemical synthesis, or both). In principle, any method that allows production of one or more templates containing a known, random, randomized, or mutagenized sequence may be used for this purpose. In one particular approach, an oligonucleotide (for example, containing random bases) is synthesized and is amplified (for example, by PCR) prior to transcription. Chemical synthesis may also be used to produce a random cassette which is then inserted into the middle of a known protein coding sequence (see, for example, chapter 8.2, Ausubel et al., Current Protocols in Molecular Biology, John Wiley & Sons and Greene Publishing Company, 1994).

An alternative to total randomization of a DNA template sequence is partial randomization, and a pool synthesized in this way is generally referred to as a "doped" pool. An example of this technique, performed on an RNA sequence, is described, for example, by Ekland et al. (Nucl. Acids Research 23:3231 (1995)). Partial randomization may be performed chemically by biasing the synthesis reactions such that each base addition reaction mixture contains an excess of one base and small amounts of each of the others; by careful control of the base concentrations, a desired mutation frequency may be achieved by this approach. Partially randomized pools may also be generated using error prone PCR techniques, for example, as described in Beaudry and Joyce (Science 257:635 (1992)) and Bartel and Szostak (Science 261:1411 (1993)).

Numerous methods are also available for generating a DNA construct beginning with a known sequence and, if desired, then creating a mutagenized DNA pool. Examples of such techniques are described in Ausubel et al. (supra, chapter 8); Sambrook et al. (Molecular Cloning: A Laboratory Manual, chapter 15, Cold Spring Harbor Press, New York, 2.sup.nd ed. (1989); Cadwell et al. (PCR Methods and Applications 2:28 (1992)); Tsang et al. (Meth. Enzymol. 267:410 (1996)); Reidhaar-Olsen et al. (Meth. Enzymol. 208:564 (1991)); and Ekland and Bartel (Nucl. Acids. Res. 23:3231 (1995)). Random sequences may also be generated by the "shuffling" technique outlined in Stemmer (Nature 370: 389 (1994)). Finally, a set of two or more homologous genes can be recombined in vitro to generate a starting library (Crameri et al. Nature 391:288-291 (1998)).

### 5.11 GENERATION OF RNA

As noted above, the RNA portion is generated by in vitro transcription of a DNA template. In one preferred approach, T7 polymerase is used to enzymatically generate the RNA strand. Transcription is generally performed in the same volume as the PCR reaction (PCR DNA derived from a 100 µml reaction is used for 100 µml of transcription). Other appropriate RNA polymerases for this use include, without limitation, the SP6, T3 and E. coli RNA polymerases (described, for example, in Ausubel et al. (supra, chapter 3). In addition, the synthesized RNA may be, in whole or in part, modified RNA. In one particular example, phosphorothioate RNA may be produced (for example, by T7 transcription) using modified ribonucleotides and standard techniques. Such modified RNA provides the advantage of being nuclease stable. Full length RNA samples are then purified from transcription reactions as previously described using urea PAGE followed by desalting on NAP-25 (Pharmacia) (Roberts and Szostak, Proc. Natl. Acad. Sci. USA 94:12297-12302 (1997)).

### 5.12 GENERATION AND RECOVERY OF DNA-RNA FUSIONS

To generate DNA-RNA fusions, any in vitro or in situ transcription system may be utilized. In principle, however, any transcription system which allows formation of a DNA-RNA fusion and which does not significantly degrade the RNA portion of the fusion is useful in the invention. In addition, to reduce RNA degradation in any of these systems, degradation-blocking antisense oligonucleotides may be included in the transcription reaction mixture; such oligonucleotides specifically hybridize to and cover sequences within the RNA portion of the molecule that trigger degradation.

In one example, any number of eukaryotic transcription systems are available for use.

Once generated, DNA-RNA fusions may be recovered from the transcription reaction mixture by any standard technique of DNA or RNA purification. Purification may also be based upon the RNA portion of the fusion; techniques for such purification are described, for example in Ausubel et al. (supra, chapter 4).

### 5.12 SELECTION OF THE DESIRED DNA-RNA FUSION

Selection of a desired DNA-RNA fusion may be accomplished by any means available to selectively partition or isolate a desired fusion from a population of candidate fusions. Examples of isolation techniques include, without limitation, catalytic activity, particular effect in an activity assay, selective binding, binding specificity, for example, to a binding partner which is directly or indirectly immobilized on a column, bead, membrane, or other solid support. The first of these techniques makes use of an immobilized selection motif which can consist of any type of molecule to which binding is possible. Selection may also be based upon the use of substrate molecules attached to an affinity label (for example, substrate-biotin) which react with a candidate molecule, or upon any other type of interaction with a fusion molecule. In addition, proteins may be selected based upon their catalytic activity; according to that particular technique, desired molecules are selected based upon their ability to link a target molecule to themselves, and the functional molecules are then isolated based upon the presence of that target. Selection schemes for isolating novel or improved aptamers using this same approach or any other functional selection are enabled by the present disclosure.

In addition, if enrichment steps targeting the same portion of the fusion (for example, the RNA portion) are repeated, different binding partners are preferably utilized. In one particular example described herein, a population of molecules is enriched for desired fusions by first using a binding partner specific for the DNA portion of the fusion and then, in two sequential steps, using two different binding partners, both of which are specific for the RNA portion of the fusion. Again, these complexes may be separated from sample components by any standard separation technique including, without limitation, column affinity chromatography, or centrifugation.

Selection and/or screening for reaction products with desired activities (such as catalytic activity, binding affinity, binding specificity or a particular effect in an activity assay) might be performed according to any standard protocol. Since minute quantities of DNA can be amplified by PCR, these selections can thus be conducted on a scale of this magnitude allowing a truly broad search for desired activities, both economical and efficient.

The display library can be selected or partitioned for binding to a target molecule. In this context, selection or partitioning means any process whereby a library member bound to a target molecule is separated from library members not bound to target molecules. Selection can be accomplished by various methods known in the art. In most applications, binding to a target molecule preferable is selective, such that the binding to the target is favored over other binding events. Ultimately, a binding molecule identified using the present invention may be useful as a therapeutic reagent and/or diagnostic agent.

The selection strategy can be carried out to allow selection against almost any target. Importantly, the selection strategy does not require any detailed structural information about the target molecule or about the members of the display library. The entire process is driven by the binding affinities and specificities involved in library members binding to a given target molecule.

Selected library members can easily be identified through their encoding nucleic acid, using standard molecular biology. The present disclosure broadly permits identifying binding molecules for any known target molecule. In addition, novel unknown targets can be discovered by isolating binding molecules of selected library members and use these for identification and validation of a target molecule.

Selection of binding molecules from a display library can be performed in any format to identify binding library members. Binding selection typically involves immobilizing the desired target molecule, adding the display library, allowing binding, and removing nonbinders/weak-binders by washing. The enriched library remaining bound to the target may be eluted with, for example acid, chaotropic salts, heat, competitive elution with known ligand, high salt, base, proteolytic release of target, enzymatic release of nucleic acids. In certain examples, the eluted library members are subjected to more rounds of binding and elution, using the same or more stringent conditions or using a different binding format, which will increase the enrichment. In other examples, the binding library members are not eluted from the target. To select for library members that bind to a protein expressed on a cell surface, such as an ion channel or a transmembrane receptor, the cells themselves can be used as selection agents. A selection procedure can also involve selection for binding to cell surface receptors that are internalized so that the receptor together with the binding molecule passes into the cytoplasm, nucleus, or other cellular compartment, such as the Golgi or lysosomes. Isolation of the compartment in question leads to partitioning of library members being internalized from non-internalized library members (Hart et al., J Biol Chem, 269, 12468-74, 1994). A selection procedure may also involve in vivo selection. The enriched library's nucleic acid portion may be amplified by, for example PCR, leading to many orders of amplification, allowing identification by e.g. cloning and DNA sequencing.

According to a specific example for affinity selection, a library of reaction products resulting from a specific member is contacted with a target under binding conditions. If one or more of the formed chemical compounds have affinity towards the target a binding will result. In a subsequent step, binding library members or a nucleic acid derived therefrom are partitioned. The nucleic acid attached to the formed chemical compound is subsequently amplified by e.g. PCR to produce multiple copies of the nucleic acid, which codes for the synthesis history of the compound displaying the desired affinity. The amplified nucleic acid can be sequenced by a number of well-known techniques to decode which chemical groups that have participated in the formation of the successful compound. Alternatively, the amplified nucleic acid can be used for the formation of a next generation library.

### 5.13 COMBINATION OF DNA DISPLAY WITH mRNA DISPLAY

As discussed above, DNA which forms the template, allows for displaying of RNA, in one example, the RNA is RNA aptamer. RNA aptamer library can be displayed on beads, as described above. Bridging the gap between particle display and RNA display means that proteins, such as antibodies or enzymes, can be selected for on bead particles, which is not possible without the presently disclosed DNA display technology. This is illustrated in FIG. 5. This allows for fluorescence-activated cell sorting (FACS) of RNA aptamers, possibly yielding binding affinities 1000 times stronger than previous RNA aptamer selection methods.

DNA display as disclosed herein connects the two other techniques: particle display and mRNA display as disclosed in U.S. Patent No. 6,261,804. mRNA display consists of an in vitro or in situ transcription/translation protocol that generates protein covalently linked to the 3' end of its own mRNA, i.e., an RNA-protein fusion. This is accomplished by synthesis and in vitro or in situ translation of an mRNA molecule with a peptide acceptor attached to its 3' end. One preferred peptide acceptor is puromycin, a nucleoside analog that adds to the C-terminus of a growing peptide chain and terminates translation. In one preferred design, a DNA sequence is included between the end of the message and the peptide acceptor which is designed to cause the ribosome to pause at the end of the open reading frame, providing additional time for the peptide acceptor (for example, puromycin) to accept the nascent peptide chain before hydrolysis of the peptidyl-tRNA linkage.

If desired, the resulting RNA-protein fusion allows repeated rounds of selection and amplification because the protein sequence information may be recovered by reverse transcription and amplification (for example, by PCR amplification as well as any other amplification technique, including RNA-based amplification techniques such as 3SR or TSA). The amplified nucleic acid may then be transcribed, modified, and in vitro or in situ translated to generate mRNA-protein fusions for the next round of selection. The ability to carry out multiple rounds of selection and amplification enables the enrichment and isolation of very rare molecules, e.g., one desired molecule out of a pool of 10¹⁵ members. This in turn allows the isolation of new or improved proteins which specifically recognize virtually any target or which catalyze desired chemical reactions.

Accordingly, in one example, provided herein is particle display-DNA display-mRNA display system. Provided herein is a method of preparing a protein library comprising: (i) providing a population of DNA display coding strands, each of which comprises a forward primer site, a T7RNA polymerase promoter, a translation start codon, a coding region and a reverse primer binding site; (ii) providing forward primer conjugated beads; (iii) annealing each of the DNA display coding strand to the forward primer conjugated bead; (iv) extending the primer on the forward primer conjugated bead by PCR in the presence of DNA polymerase to form a bead comprising a double stranded DNA display template; (v) Denaturing the double stranded DNA display template to form a bead comprising a single stranded DNA display template strand; (vi) annealing a NTP-PEG-DNA conjugate comprising a reverse primer with the single stranded DNA display template strand; (vii) extending the reverse primer of the NTP-PEG-DNA conjugate by PCR in the presence of DNA polymerase to form a bead comprising a double stranded DNA display template with PEG-NTP; (viii) in vitro transcription of the DNA display template on beads comprising the double stranded DNA display template producing DNA-RNA fusion comprising a DNA portion and a RNA portion; and (ix) in vitro translating the RNA portion to produce a population of RNA-protein fusions. The product of an DNA display in combination with RNA display is a DNA-RNA-protein fusion (FIG. 5, Panel 3).

In one example, the RNA-protein fusion comprises an mRNA linked to the protein via a puromycin linkage.

In one example, the forward primer conjugated beads are formed by an amino bond formed between carboxylic acid functionalized beads and an amine functionalized forward primer.

Also provided herein is a method of selecting a target protein comprising: (i) providing a DNA coding strand comprising a forward primer site, a T7RNA polymerase promoter, a translation start codon, a random library region and a reverse primer binding site; (ii) Providing a forward primer conjugated bead (an amino bond formed between carboxylic acid functionalized beads and an amine functionalized forward primer); (iii) Annealing the DNA display library coding strand to the forward primer conjugated bead; (iv) Iterating cycles of PCR in the presence of DNA polymerase to extend the primer on the forward primer conjugated bead to form a bead comprising a double stranded DNA display template; (v) denaturing the double stranded DNA display template to form a bead comprising a single stranded DNA display template strand; (vi) annealing a NTP-PEG-DNA conjugate comprising a reverse primer with the single stranded DNA display template strand; (vii) extending the reverse primer of the NTP-PEG-DNA conjugate by PCR in the presence of DNA polymerase to form a bead comprising a double stranded DNA display template with PEG-NTP; (viii) in vitro transcription of the DNA display template on beads comprising the double stranded DNA display template producing DNA-RNA fusion comprising a DNA portion and a RNA portion; (ix) in vitro translating the RNA portion to produce a population of RNA-protein fusions, thereby producing a protein library; and (x) selecting a desired RNA-protein fusion based on fusion binding or activity, thereby selecting said desired protein and said nucleic acid encoding said protein.

In one example, the desired protein is antibodies, fragments of antibodies, aptazymes or enzymes.

In one example, the desired protein is selected by FACs.

In one example, the nucleotide triphosphate (aa-NTP) is adenosine triphosphate (ATP), guanosine triphosphate (GTP), cytidine triphosphate (CTP), 5-methyluridine triphosphate (m5UTP), uridine triphosphate (UTP), any unnatural nucleoside triphosphate.

### 5.14 METHOD OF USING THE DNA DISPLAY

DNA display via the DNA display template can be used to capture RNA after transcription. Applications of this capture can be research based, as in the quantification of transcription products, or more applied, as in correlating genotype (DNA) to phenotype (RNA) for in vitro evolution of RNA aptamers, DNA aptamers, peptide nucleic acids (PNAs), and xeno nucleic acids (XNAs). The selection systems of the present disclosure have commercial applications in any area where RNA technology is used to solve therapeutic, diagnostic, or industrial problems. This selection technology is useful for improving or altering existing RNA as well as for isolating new aptamers with desired functions. These RNA may be naturally-occurring sequences, may be altered forms of naturally-occurring sequences, or may be partly or fully synthetic sequences. In addition, these methods may also be used to isolate or identify useful nucleic acid or small molecule targets.

Isolation of Novel Binding Reagents - in one particular application, the DNA-RNA fusion technology described herein is useful for the isolation of DNA aptamers, RNA aptamers, peptide nucleic acids and xeno nucleic acids with specific binding (for example, ligand binding) properties. Aptamers exhibiting highly specific binding interactions may be used as non-antibody recognition reagents, allowing DNA-RNA fusion technology to circumvent traditional monoclonal antibody technology. Antibody-type reagents isolated by this method may be used in any area where traditional antibodies are utilized, including diagnostic and therapeutic applications.

Nucleic acids have sufficient capacity for forming a variety of two- and three-dimensional structures and sufficient chemical versatility available within their monomers to act as ligands (form specific binding pairs) with virtually any chemical compound, whether monomeric or polymeric. Molecules of any size or composition can serve as targets. A suitable method for generating an aptamer to a target of interest is the SELEX method which involves selection from a mixture of candidate oligonucleotides and step-wise iterations of binding, partitioning and amplification, using the same general selection scheme, to achieve virtually any desired criterion of binding affinity and selectivity. Starting from a mixture of nucleic acids, preferably comprising a segment of randomized sequence, the SELEX.TM. method includes steps of contacting the mixture with the target under conditions favorable for binding, partitioning unbound nucleic acids from those nucleic acids which have bound specifically to target molecules, dissociating the nucleic acid-target complexes, amplifying the nucleic acids dissociated from the nucleic acid-target complexes to yield a ligand-enriched mixture of nucleic acids, then reiterating the steps of binding, partitioning, dissociating and amplifying through as many cycles as desired to yield highly specific high affinity nucleic acid ligands to the target molecule. In certain examples, the PCR may undergo 2-30 cycles, 2-10, 10-20, and 20-30 cycles.

A variety of nucleic acid primary, secondary and tertiary structures are known to exist. The structures or motifs that have been shown most commonly to be involved in non-Watson-Crick type interactions are referred to as hairpin loops, symmetric and asymmetric bulges, pseudoknots and myriad combinations of the same. Almost all known cases of such motifs suggest that they can be formed in a nucleic acid sequence of no more than 30 nucleotides. For this reason, it is often preferred that SELEX procedures with contiguous randomized segments be initiated with nucleic acid sequences containing a randomized segment of between about 20-50 nucleotides. In one example, the DNA coding region has 20-50 nucleotides. In one example, the DNA coding region has 20-30 nucleotides. In one embodiment, the DNA coding region has 30-50 nucleotides. In one example, the DNA coding region has more than 50 nucleotides.

Isolation of New Catalysts - the present disclosure may also be used to select new catalytic aptamers. In vitro selection and evolution can be used for the isolation of novel catalytic RNA and DNA. In one particular example of this approach, a catalyst may be isolated indirectly by selecting for binding to a chemical analog of the catalyst's transition state. In another particular example, direct isolation may be carried out by selecting for covalent bond formation with a substrate (for example, using a substrate linked to an affinity tag) or by cleavage (for example, by selecting for the ability to break a specific bond and thereby liberate catalytic members of a library from a solid support).

### 5.14.1 METHOD TO CAPTURE RNA AFTER TRANSCRIPTION

By assembling a DNA display template, which comprises of the template DNA ligated to an NTP, the desired conditions for RNA capture are created. Upon translation of a DNA display template the ligated NTP will be inserted into the newly forming RNA strand giving a covalent link between the RNA and the DNA which encodes it (figure 5B). In this way RNA capture can be achieved.

### 5.14.2 QUANTIFICATION OF TRANSCRIPTION PRODUCTS

Genotype phenotype linkage involves the retaining of information after some conversion event. This information can be a genetic sequence or spatial in nature, such as a coupled ligation to beads. Having performed DNA display on a DNA template the resultant RNA DNA fusion can undergo mRNA display. In mRNA display puromycin is linked to the end of the RNA strand and during translation, is inserted into the newly forming polypeptide. This protein is then coupled to the beads via the RNA and the DNA which encodes it (Figure 5C).

Quantifying the degree to which a gene is transcribed poses a difficult scientific problem. Most methods involve the modification of the gene or its environment to a substantial degree, such as inserting a reporter transcript or cloning the gene out of its genome for expression.

A PCR primer which complements the end of the gene in question can be conjugated to an NTP via a PEG linker. After inserting this modified primer DNA into the genome, after transcription of the gene it will have the RNA which encodes it covalently attached. A site specific nuclease digestion and analysis of bands on PAGE will show a shift in mass of the gene in question due to the extra mass of the displayed RNA. In this way the transcription of a gene can be quantified.

### 5.15 USE OF DNA DISPLAY IN COMBINATION WITH mRNA DISPLAY SELECTION SYSTEM

The selection systems of the present disclosure have commercial applications in any area where protein technology is used to solve therapeutic, diagnostic, or industrial problems. This selection technology is useful for improving or altering existing proteins as well as for isolating new proteins with desired functions. These proteins may be naturally-occurring sequences, may be altered forms of naturally-occurring sequences, or may be partly or fully synthetic sequences. In addition, these methods may also be used to isolate or identify useful nucleic acid or small molecule targets.

**Isolation of Novel Binding Reagents.** In one particular application, the DNA-RNA-protein fusion technology described herein is useful for the isolation of proteins with specific binding (for example, ligand binding) properties. Proteins exhibiting highly specific binding interactions may be used as non-antibody recognition reagents, allowing DNA-RNA-protein fusion technology to circumvent traditional monoclonal antibody technology. Antibody-type reagents isolated by this method may be used in any area where traditional antibodies are utilized, including diagnostic and therapeutic applications.

**Improvement of Human Antibodies.** The present disclosure may also be used to improve human or humanized antibodies for the treatment of any of a number of diseases. In this application, antibody libraries are developed and are screened in vitro, eliminating the need for techniques such as cell-fusion or phage display. In one important application, the invention is useful for improving single chain antibody libraries (Ward et al., Nature 341:544 (1989); and Goulot et al., J. Mol. Biol. 213:617 (1990)). For this application, the variable region may be constructed either from a human source (to minimize possible adverse immune reactions of the recipient) or may contain a totally randomized cassette (to maximize the complexity of the library). To screen for improved antibody molecules, a pool of candidate molecules are tested for binding to a target molecule (for example, an antigen immobilized as shown in FIG. 2). Higher levels of stringency are then applied to the binding step as the selection progresses from one round to the next. To increase stringency, conditions such as number of wash steps, concentration of excess competitor, buffer conditions, length of binding reaction time, and choice of immobilization matrix are altered.

Single chain antibodies may be used either directly for therapy or indirectly for the design of standard antibodies. Such antibodies have a number of potential applications, including the isolation of anti-autoimmune antibodies, immune suppression, and in the development of vaccines for viral diseases such as AIDS.

**Isolation of New Catalysts.** The present disclosure may also be used to select new catalytic proteins. In vitro selection and evolution has been used previously for the isolation of novel catalytic RNAs and DNAs, and, in the present disclosure, is used for the isolation of novel protein enzymes. In one particular example of this approach, a catalyst may be isolated indirectly by selecting for binding to a chemical analog of the catalyst's transition state. In another particular example, direct isolation may be carried out by selecting for covalent bond formation with a substrate (for example, using a substrate linked to an affinity tag) or by cleavage (for example, by selecting for the ability to break a specific bond and thereby liberate catalytic members of a library from a solid support). In another particular example the enzymatic reaction selected for can be linked to a secondary signal reaction consisting of but not restricted to colour change, fluorescence or luminescence.

This approach to the isolation of new catalysts has at least two important advantages over catalytic antibody technology (reviewed in Schultz et al., J. Chem. Engng. News 68:26 (1990)). First, in catalytic antibody technology, the initial pool is generally limited to the immunoglobulin fold; in contrast, the starting library of DNA-RNA-protein fusions may be either completely random or may consist, without limitation, of variants of known enzymatic structures or protein scaffolds. In addition, the isolation of catalytic antibodies generally relies on an initial selection for binding to transition state reaction analogs followed by laborious screening for active antibodies; again, in contrast, direct selection for catalysis is possible using a DNA-RNA-protein fusion library approach, as previously demonstrated using RNA libraries. In an alternative approach to isolating protein enzymes, the transition-state-analog and direct selection approaches may be combined.

Enzymes obtained by this method are highly valuable. For example, there currently exists a pressing need for novel and effective industrial catalysts that allow improved chemical processes to be developed. A major advantage of the disclosure is that selections may be carried out in arbitrary conditions and are not limited, for example, to in vivo conditions. The disclosure therefore facilitates the isolation of novel enzymes or improved variants of existing enzymes that can carry out highly specific transformations (and thereby minimize the formation of undesired byproducts) while functioning in predetermined environments, for example, environments of elevated temperature, pressure, or solvent concentration.

### 5.16 USE OF RNA-PROTEIN FUSIONS IN A MICROCHIP FORMAT

"DNA chips" consist of spatially defined arrays of immobilized oligonucleotides or cloned fragments of cDNA or genomic DNA, and have applications such as rapid sequencing and transcript profiling. By annealing a mixture of DNA-RNA fusions (for example, generated from a cellular DNA or RNA pool), to such a DNA chip, it is possible to generate a "RNA display chip," in which each spot corresponding to one immobilized sequence is capable of annealing to its corresponding DNA sequence in the pool of DNA-RNA fusions. By this approach, the corresponding RNA is immobilized in a spatially defined manner because of its linkage to its own DNA, and chips containing sets of DNA sequences display the corresponding set of RNA.

Such ordered displays of RNA have many uses. For example, they represent powerful tools for the identification of previously unknown interactions between RNA and other molecules. RNA display technology may be carried out using arrays of nucleic acids (including RNA, but preferably DNA) immobilized on any appropriate solid support. Exemplary solid supports may be made of materials such as glass (e.g., glass plates), silicon or silicon-glass (e.g., microchips), or gold (e.g., gold plates). Methods for attaching nucleic acids to precise regions on such solid surfaces, e.g., photolithographic methods, are well known in the art, and may be used to generate solid supports (such as DNA chips).

### 6. EXAMPLES

### 6.1

### CTP-PEG-DNA synthesis:

### Specialised reagents:

5-Aminoallylcytidine-5'-triphosphate (TriLink BioTechnologies)
5' Thiol modified DNA oligo (Integrated DNA Technologies)
Malemide, NHS Heterobifunctional PEG linker (NANOCS)

### Buffers needed:

TLC Buffer: 95% ether 5% acetone
1 X Conjugation buffer (PBS)
1.25 X Conjugation buffer (PBS)
Buffer A: 20mM Tris-HCL, pH 8.5
Buffer B: 1M NaCl, 20mM Tris-HCL, pH 8.5

Note: For best results, ensure that DNA-SH is prepared and ready to combine with NTP-PEG-NH2 in step 5.

### Oligonucleotide reduction:

1. The DNA oligo was resuspended in PBS pH 7.0, at about 1nmole/µL.
2. TCEP slurry was mixed and a volume equal to about 2x the volume of oligo was taken. The TCEP slurry was centrifuged and the supernatant removed. The TCEP slurry was then washed with PBS pH 7.0.
3. The DNA oligo was then added to the TCEP slurry and incubated at RT with constant mixing for 2 hours.
4. The TCEP slurry/DNA oligo mix was then transferred to a PALL Nanosep 100 microspin column and centrifuged for 5 min at ∼10000 rpm. The filtrate contains the reduced thiol modified DNA oligo.

### Conjugation:

1. Prepare appropriate TLC plates with lanes for crosslinker, ntp, t=0, t=30min, t=60min.
2. Combine 10uL of 100mM aa-CTP solution and 40ul of 4.6875mM Malemide-PEG3400-NHS in 1.25X PBS, pH7.2 solution.
3. Incubate reaction mixture for 1h at room temperature or 2 hours at 4°C. Monitor the reaction using TLC.
4. Remove excess CTP using G10 Sephadex micro gel filtration column.
5. Combine and mix the reduced DNA-SH and desalted NTP-PEG- in a 5X molar ratio with excess DNA-SH.
6. Incubate the reaction mixture at room temperature for 30 minutes or 2 hours at 4°C. Monitor reaction with TLC. Note: Generally, there is no harm in allowing the reaction to proceed for several hours or overnight, although usually the reaction will be complete in the specified time. To stop the conjugation reaction before completion, add buffer containing reduced cysteine at a concentration several times greater than the sulfhydryls of Protein-SH.
7. Purify using, ion exchange chromatography

### Ion exchange buffers:

Buffer A: 20mM Tris-HCL, pH 8.5
Buffer B: 1M NaCl, 20mM Tris-HCL, pH 8.5
Gradient 0-100% over 30 min
Detection 254nm for CTP and 260nm for DNA.
Fractions can then be characterised using mass spectrometry and PAGE electrophoresis.

### Chain extension of CTP-PEG-DNA to DNA template:

| Invitrogen Pfx PCR reaction mixture | |
|---|---|
| 10 X Buffer | 5µL |
| ddH₂O | 26.1µL |
| 50mM MgSO₄ | 1µL |
| NTP-PEG-DNA primer 16µM | 15µL |
| Biotinylated Template ssDNA 50µM | 1µL |
| dNTP mix 10mM | 1.5µL |
| Pfx DNA polymerase | 0.4µL |
| Total | 50µL |

### Thermal cycler program

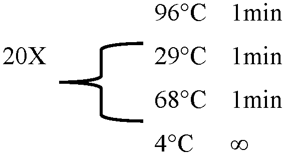

### DNA display:

Biotinylated dsDNA-PEG-CTP template was incubated with streptavidin beads for 5 minuted and washed 3 times.
Beads were then resuspended in:

| | |
|---|---|
| 100mM TEA pH 7.6 | 27 µL |
| 50mM MgCl₂ | 5 µL |
| 10mg/ml BSA | 5 µL |
| 100mM ATP | 5 µL |
| 1 unit Cytidylate kinase | 8 µL |

### And incubated at RT for 30 minutes.

The beads were then washed 3 times and resuspended in

| | |
|---|---|
| ddH₂O | 39 µL |
| 10X t4 ligase buffer | 5 µL |
| 100mM ATP | 5 µL |
| 1 unit nucleoside 5'-diphosphate kinase | 1 µL |

### And incubated at RT for 30 minutes.

The beads were then washed 3 times in ddH₂O and divided into the no transcription (-T) and transcription (+T) aliquots in the ratio of 1/3 to 2/3 respectively (one -T reaction, one +T reaction and one +T RNAse reaction).

The +T beads were then resuspended in epicentre T7 Flash Transcription Kit reaction mixture:

| | |
|---|---|
| DEPC treated ddH₂O | 6.3 µL |
| 10 X transcription buffer | 2 µL |
| 100mM ATP | 1.8 µL |
| 100mM GTP | 1.8 µL |
| 100mM CTP | 1.8 µL |
| 100mM UTP | 1.8 µL |
| 100mM DTT | 2 µL |
| RNAse inhibitor | 0.5 µL |
| T7 RNA polymerase | 2 µL |
| Total volume | 20 µL |

### And incubated at 37°C for 30 minutes.

The +T aliquot was then divided in two (one +T reaction and one +T and RNAse reaction). The samples -T, +T and +T RNAse were resuspended in 19 µL of RNAse buffer (ImM MgCl2, 0.5mM CaC12, 10mM Tris pH7.5). The -T and +T had 1 µL of ddH₂O added and the +T RNAse sample had 1µL of 10µg/ml RNAse A added. All samples were then incubated at 37°C for 15 minutes before being washed 3 times in ddH2O. The samples -T, +T and +T RNAse were then eluted from the beads by treatment in 3uL of 95% formamide at 90°C for 2 minutes. Samples were then loaded onto a 7M Urea 10% PAGE gel for analysis (Figure 4).

While the foregoing invention has been described in some detail for purposes of clarity and understanding, it will be appreciated by one skilled in the art, from a reading of the disclosure, that various changes in form and detail can be made without departing from the true scope of the invention in the appended claims.

### REFERENCES

1. Nucleic acid ligands US5696249 A
2. Yeast cell surface display of proteins and uses thereof US6699658 B1
3. Expression of proteins on bacterial surface US5348867 A
4. Selection of proteins using RNA-protein fusions US6261804 B1
5. Ribosome complexes as selection particles for in vitro display and evolution of proteins US6620587 B1
6. Phage display US8685893 B2
7. Expression of proteins on bacterial surface US5348867 A
8. In vitro peptide expression library US8679781 B2
9. Oligonucleotide-linked magnetic particles and uses thereof US5512439 A
10. Benatuil, L., et al. (2010). "An improved yeast transformation method for the generation of very large human antibody libraries." Protein Engineering Design and Selection 23(4): 155-159.
11. Coomber, D., et al. (2004). In vitro peptide expression libraray.
12. Earhart, C. F., et al. (1993). Expression of proteins on bacterial surface.
13. He, M. and M. J. Taussig (1998). Ribosome complexes as selection particles for in vitro display and evolution of proteins.
14. Liu, R., et al. (2000). Selection of proteins using rna-protein fusions.
15. Osborne, S. E. and A. D. Ellington (1997). "Nucleic acid selection and the challenge of combinatorial chemistry." Chemical Reviews 97(2): 349-370.
16. Scott, J. K. and G. P. Smith (1990). "Searching for peptide ligands with an epitope library." Science 249(4967): 386-390.
17. Sidhu, S. S., et al. (2012). Phage display.
18. Van Antwerp, J. J. and K. D. Wittrup (2000). "Fine affinity discrimination by yeast surface display and flow cytometry." Biotechnology progress 16(1): 31-37.
19. Wang, J., et al. (2014). "Particle Display: A Quantitative Screening Method for Generating High-Affinity Aptamers." Angewandte Chemie International Edition 53(19): 4796-4801.
20. Wilson, D. S., et al. (2001). "The use of mRNA display to select high-affinity protein-binding peptides." Proceedings of the National Academy of Sciences 98(7): 3750-3755.
21. Wittrup, K. D., et al. (2004). Yeast cell surface display of proteins and uses thereof.

## Claims

1. A method of producing an NTP-PEG-DNA conjugate comprising:
(i) conjugating aminoallyl nucleoside triphosphate (aa-NTP) with a N-hydroxysuccinimide (NHS)-PEG-maleimide (NHS-PEG) forming NTP-PEG-maleimide (NTP-PEG);
(ii) conjugating a DNA oligonucleotide functionalized with a reduced thiol (thiol-DNA) with the maleimide on the NTP-PEG forming NTP-PEG-DNA; and
(iii) purifying the NTP-PEG-DNA conjugate.

2. A method as claimed in claim 1, wherein the thiol-DNA is
(a) a DNA library comprising DNA polynucleotides functionalized with the reduced thiol; or
(b) a population of DNA polynucleotides functionalized with a reduced thiol,
and wherein the library or population of thiol-DNA is conjugated with the maleimide on the NTP-PEG forming NTP-PEG-DNA; and the library or population of NTP-PEG-DNA conjugates is purified.

3. A method as claimed in claim 1, wherein
(a) the aa-NTP is aminoallyl cytidine triphosphate; or
(b) the NHS-PEG is N-hydroxysuccinimide maleimide; or
(c) the reduced thiol is a reduced 5' C6 S-S thiol modified DNA oligonucleotide; or
(d) the NTP-PEG-DNA conjugate is a CTP-PEG-DNA conjugate.

4. A method as claimed in claim 1, wherein the DNA is a reverse primer.

5. A method of producing a DNA display library comprising:
(i) providing a population of DNA coding strands, each of which comprises a forward primer site, a T7RNA polymerase promoter, a DNA coding region and a reverse primer binding site;
(ii) annealing each DNA coding strand to a forward primer;
(iii) extending the primer in the presence of DNA polymerase to form a population of double stranded DNA display templates;
(iv) denaturing the population of double stranded DNA display templates to form a population of single stranded DNA display template strands;
(v) annealing a NTP-linker-DNA conjugate comprising a reverse primer to each of the single stranded DNA display template strand;
(vi) extending the reverse primer of the NTP-linker-DNA conjugates by PCR in the presence of DNA to form a population of double stranded DNA display templates comprising a linker-NTP.

6. A method as claimed in claim 5, wherein the linker has a length of 100-200 angstrom, 200-250 angstrom, 250-280 angstrom, 280-300 angstrom, 300-400 angstrom, or a length of 200-300 angstrom.

7. A method as claimed in claim 5, wherein the linker does not comprise polynucleotides.

8. A method as claimed in claim 5, wherein the linker is PEG and the method further comprises the step of:
(vii) *in vitro* transcription of the DNA display template of the double stranded DNA display template, so as to prepare a library comprising RNA aptamers.

9. A method as claimed in claim 5, wherein the linker is PEG and the forward primers are conjugated to beads so that DNA display template coated beads are produced.

10. A method as claimed in claim 5, wherein the linker is PEG and the forward primers are conjugated to beads, and the method further comprises (vii) *in vitro* transcription of the DNA display templates on beads comprising the double stranded DNA display template, so as to prepare a library comprising RNA aptamers.

11. A method as claimed in any of claims 5 to 10, wherein the NTP-linker-DNA or NTP-PEG-DNA conjugate is produced by a method comprising the steps:
(i) conjugating aminoallyl nucleoside triphosphate (aa-NTP) with a N-hydroxysuccinimide (NHS)-PEG-maleimide (NHS-PEG) forming NTP-PEG-maleimide (NTP-PEG);
(ii) conjugating a DNA oligonucleotide functionalized with a reduced thiol (thiol-DNA) with the maleimide on the NTP-PEG forming NTP-PEG-DNA; and
(iii) purifying the NTP-PEG-DNA conjugate.

12. A method as claimed in claim 9 or claim 10, wherein the PCR is emulsion PCR, where each bead displays multiple copies of a single coding strand sequence; preferably wherein the emulsion is broken and the double stranded DNA on the beads is denatured to single stranded DNA.

13. A method as claimed in claim 5, wherein each DNA display coding strand in the population further comprises a translation start codon, the linker is PEG and the forward primers are conjugated to beads; and wherein the method further comprises the steps of
(vii) *in vitro* transcription of the DNA display template on beads comprising the double stranded DNA display template producing DNA-RNA fusion comprising a DNA portion and a RNA portion; and
(viii) *in vitro* translating the RNA portion to produce a population of RNA-protein fusions, thereby producing a protein library.

14. A method as claimed in claim 13, wherein the DNA coding region in each coding strand is a random library region, and the method further comprises the step of:
(ix) selecting a desired RNA-protein fusion based on fusion binding or activity, thereby selecting a desired target protein and said nucleic acid encoding said protein.

15. A method as claimed in claim 13 or claim 14, wherein the RNA-protein fusion comprises an mRNA linked to the protein via a puromycin linkage.

16. A method as claimed in any of claims 9 to 14, wherein the forward primer conjugated bead or beads are formed by an amino bond formed between carboxylic acid functionalized beads and an amine functionalized forward primer.

17. A method as claimed in claim 14 wherein:
(a) the desired protein is an antibody, an antibody fragment, an aptazyme or an enzyme; or
(b) the desired protein is selected by FACS.

18. A method as claimed in claim 14, wherein:
(a) the nucleotide triphosphate (aa-NTP) is adenosine triphosphate (ATP), guanosine triphosphate (GTP), cytidine triphosphate (CTP), 5-methyluridine triphosphate (m5UTP), uridine triphosphate (UTP), or an unnatural nucleoside triphosphate; or
(b) the RNA polymerase substrate is an alternate linking chemistry added for attachment to the PEG; or
(c) the PEG is PEG3100, PEG 3200, PEG3300, PEG3400, PEG3500 or PEG3600.

19. An NTP-PEG-DNA conjugate comprising a DNA polynucleotide and a nucleoside triphosphate (NTP), wherein the DNA polynucleotide and the NTP are conjugated via PEG.

## Patentansprüche

1. Verfahren zur Erzeugung eines NTP-PEG-DNA-Konjugats, wobei das Verfahren folgendes umfasst:
(i) Konjugieren eines Aminoallyl-Nucleosid-Triphosphats (aa-NTP) mit einem N-Hydroxysuccinimid (NHS)-PEG-Maleimid (NHS-PEG), das ein NTP-PEG-Maleimid (NTP-PEG) bildet;
(ii) Konjugieren eines mit einem reduzierten Thiol (Thiol-DNA) funktionalisierten DNA-Oligonucleotids mit dem Maleimid an der NTP-PEG bildenden NTP-PEG-DNA; und
(iii) Aufreinigen des NTP-PEG-DNA-Konjugats.

2. Verfahren nach Anspruch 1, wobei die Thiol-DNA folgendes ist:
(a) eine DNA-Bibliothek, die mit dem reduzierten Thiol funktionalisierte DNA-Polynucleotide umfasst; oder
(b) eine Population von mit einem reduzierten Thiol funktionalisierten DNA-Polynucleotiden, und wobei die Bibliothek oder Population von Thiol-DNA konjugiert wird mit dem Maleimid an dem NTP-PEG-DNA bildenden NTP-PEG; und wobei die Bibliothek oder Population von NTP-PEG-DNA-Konjugaten aufgereinigt wird.

3. Verfahren nach Anspruch 1, wobei
(a) das aa-NTP Aminoallyl-Cytidin-Triphosphat ist; oder
(b) das NHS-PEG N-Hydroxysuccinimid-Maleimid ist; oder
(c) das reduzierte Thiol ein reduziertes 5' C6 S-S-Thiol modifiziertes DNA-Oligonucleotid ist; oder
(d) das NTP-PEG-DNA-Konjugat ein CTP-PEG-DNA-Konjugat ist.

4. Verfahren nach Anspruch 1, wobei die DNA ein Rückwärtsprimer ist.

5. Verfahren zur Erzeugung einer DNA-Display-Bibliothek, wobei das Verfahren folgendes umfasst:
(i) Bereitstellen einer Population von DNA-Codierungssträngen, von denen jeder eine Vorwärtsprimerstelle, einen T7RNA-Polymerase-Promotor, einen DNA-Codierungsbereich und eine Rückwärtsprimer-Bindungsstelle umfasst;
(ii) Anlagern jedes DNA-Codierungsstrangs an einen Vorwärtsprimer;
(iii) Erweitern des Primers in Gegenwart von DNA-Polymerase, um eine Population doppelsträngiger DNA-Display-Vorlagen zu bilden;
(iv) Denaturieren der Population doppelsträngiger DNA-Display-Vorlagen, um eine Population einzelsträngiger DNA-Display-Vorlagenstränge zu bilden;
(v) Anlagern eines NTP-Linker-DNA-Konjugats, das einen Rückwärtsprimer umfasst, an jeden der einzelsträngigen DNA-Display-Vorlagenstränge;
(vi) Erweitern des Rückwärtsprimers der NTP-Linker-DNA-Konjugate durch PCR in Gegenwart einer DNA, um eine Population doppelsträngiger DNA-Display-Vorlagen, die eine Linker-NTP umfassen, zu bilden.

6. Verfahren nach Anspruch 5, wobei der Linker eine Länge von 100-200 Angstrom, 200-250 Angstrom, 250-280 Angstrom, 280-300 Angstrom, 300-400 Angstrom oder eine Länge von 200-300 Angstrom aufweist.

7. Verfahren nach Anspruch 5, wobei der Linker keine Polynucleotide umfasst.

8. Verfahren nach Anspruch 5, wobei der Linker PEG ist, und wobei das Verfahren ferner den folgenden Schritt umfasst:
(vii) *in* vitro-Transkription der DNA-Display-Vorlage der doppelsträngigen DNA-Display-Vorlage, um eine Bibliothek zu erstellen, die RNA-Aptamere umfasst.

9. Verfahren nach Anspruch 5, wobei der Linker PEG ist, und wobei die Vorwärtsprimer an Kügelchen konjugiert sind, so dass mit DNA-Display-Vorlage überzogene Kügelchen erzeugt werden.

10. Verfahren nach Anspruch 5, wobei der Linker PEG ist, und wobei die Vorwärtsprimer an Kügelchen konjugiert sind, und wobei das Verfahren ferner folgendes umfasst: (vii) *in vitro*-Transkription der DNA-Display-Vorlage der doppelsträngigen DNA-Display-Vorlage, um eine Bibliothek zu erstellen, die RNA-Aptamere umfasst.

11. Verfahren nach einem der Ansprüche 5 bis 10, wobei die NTP-Linker-DNA oder das NTP-PEG-DNA-Konjugat durch ein Verfahren erzeugt wird, das die folgenden Schritte umfasst:
(i) Konjugieren von Aminoallyl-Nucleosid-Triphosphat (aa-NTP) mit einem N-Hydroxysuccinimid (NHS)-PEG-Maleimid (NHS-PEG), das NTP-PEG-Maleimid (NTP-PEG) bildet;
(ii) Konjugieren eines mit einem reduzierten Thiol (Thiol-DNA) funktionalisierten DNA-Oligonucleotids mit dem Maleimid an dem die NTP-PEG-DNA bildenden NTP-PEG; und
(iii) Aufreinigen des NTP-PEG-DNA-Konjugats.

12. Verfahren nach Anspruch 9 oder Anspruch 10, wobei die PCR Emulsions-PCR ist, wobei jedes Kügelchen mehrere Kopien einer einzelnen Codierungsstrangsequenz anzeigt; wobei die Emulsion gebrochen wird, und wobei die doppelsträngige DNA an den Kügelchen in einzelsträngige DNA denaturiert wird.

13. Verfahren nach Anspruch 5, wobei jeder DNA-Display-Codierungsstrang in der Population ferner ein Translationsanfangs-Codon umfasst, wobei der Linker PEG, und wobei die Vorwärtsprimer an Kügelchen konjugiert sind; und wobei das Verfahren ferner die folgenden Schritte umfasst:
(vii) *in vitro*-Transkription der DNA-Display-Vorlage an Kügelchen, umfassend die doppelsträngige DNA-Display-Vorlage, wobei eine DNA-RNA-Fusion erzeugt wird, die einen DNA-Teil und einen RNA-Teil umfasst; und
(viii) *in vitro*-Translation des RNA-Teils, um eine Population von RNA-Protein-Fusionen zu erzeugen, wodurch eine Proteinbibliothek erzeugt wird.

14. Verfahren nach Anspruch 13, wobei der DNA-Codierungsbereich in jedem Codierungsstrang ein willkürlicher Bibliotheksbereich ist, und wobei das Verfahren ferner den folgenden Schritt umfasst:
(ix) Auswählen einer gewünschten RNA-Protein-Fusion auf der Basis der Fusionsbindung oder Aktivität, wodurch ein gewünschtes Zielprotein ausgewählt wird und die für das Protein codierende Nucleinsäure.

15. Verfahren nach Anspruch 13 oder Anspruch 14, wobei die RNA-Protein-Fusion eine mRNA umfasst, die über eine Puromycin-Verknüpfung mit dem Protein verknüpft ist.

16. Verfahren nach einem der Ansprüche 9 bis 14, wobei das bzw. die mit Vorwärtsprimer konjugierte(n) Kügelchen durch eine Aminobindung gebildet werden, die zwischen mit Carbonsäure funktionalisierten Kügelchen und mit Amin funktionalisiertem Vorwärtsprimer gebildet wird.

17. Verfahren nach Anspruch 14, wobei:
(a) das gewünschte Protein ein Antikörper, ein Antikörperfragment, ein Aptazym oder ein Enzym ist; oder
(b) das gewünschte Protein durch FACS ausgewählt wird.

18. Verfahren nach Anspruch 14, wobei:
(a) das Nucleotid-Triphosphat (aa-NTP) ein Adenosin-Triphosphat (ATP), Guanosin-Triphosphat (GTP), Cytidin-Triphosphat (CTP), 5-Methyluridin-Triphosphat (m5UTP), Uridin-Triphosphat (UTP) oder ein unnatürliches Nucleosid-Triphosphat ist; oder
(b) das RNA-Polymerase-Substrat eine zur Anhaftung an das PEG hinzugefügte alternative Verknüpfungschemie ist; oder
(c) das PEG PEG3100, PEG3200, PEG3300, PEG3400, PEG3500 oder PEG3600 ist.

19. NTP-PEG-DNA-Konjugat, das ein DNA-Polynucleotid und ein Nucleosid-Triphosphat (NTP) umfasst, wobei das DNA-Polynucleotid und das NTP über PEG konjugiert sind.

## Revendications

1. Procédé de production d'un conjugué NTP-PEG-ADN comprenant :
(i) la conjugaison d'un aminoallyl nucléoside triphosphate (aa-NTP) avec un N-hydroxysuccinimide (NHS)-PEG-maléimide (NHS-PEG) formant un NTP-PEG-maléimide (NTP-PEG) ;
(ii) la conjugaison d'un oligonucléotide d'ADN fonctionnalisé avec un thiol réduit (thiol-ADN) avec le maléimide sur le NTP-PEG formant un NTP-PEG-ADN ;
et
(iii) la purification du conjugué NTP-PEG-ADN.

2. Procédé selon la revendication 1, dans lequel le thiol-ADN est
(a) une banque d'ADN comprenant de polynucléotides d'ADN fonctionnalisés avec le thiol réduit ; ou
(b) une population de polynucléotides d'ADN fonctionnalisés avec un thiol réduit, et dans lequel la banque ou population de thiol-ADN est conjuguée avec le maléimide sur le NTP-PEG formant un NTP-PEG-ADN ; et la banque ou population de conjugués NTP-PEG-ADN est purifiée.

3. Procédé selon la revendication 1, dans lequel
(a) l'aa-NTP est un aminoallyl cytidine triphosphate ; ou
(b) le NHS-PEG est le N-hydroxysuccinimide maléimide ; ou
(c) le thiol réduit est un oligonucléotide d'ADN à modification 5' C6 S-S thiol réduit ; ou
(d) le conjugué NTP-PEG-ADN est un conjugué CTP-PEG-ADN.

4. Procédé selon la revendication 1, dans lequel l'ADN est une amorce inverse.

5. Procédé de production d'une banque d'affichage d'ADN comprenant :
(i) la fourniture d'une population de brins codants d'ADN, chacun desquels comprenant un site d'amorce avant, un promoteur polymérase T7ARN, une région codant l'ADN et un site de liaison d'amorce inverse ;
(ii) l'hybridation de chaque brin codant d'ADN à une amorce avant ;
(iii) l'extension de l'amorce en présence d'ADN polymérase afin de former une population de matrices d'affichage d'ADN bicaténaires ;
(iv) la dénaturation de la population de matrices d'affichage d'ADN bicaténaires afin de former une population de brins de matrice d'affichage d'ADN monocaténaires ;
(v) l'hybridation d'un conjugué NTP-lieur-ADN comprenant une amorce inverse à chacun des brins de matrice d'affichage d'ADN monocaténaires ;
(vi) l'extension de l'amorce inverse des conjugués NTP-lieur-ADN par PCR en présence d'ADN afin de former une population de matrices d'affichage d'ADN bicaténaires comprenant un lieur-NTP.

6. Procédé selon la revendication 5, dans lequel le lieur a une longueur de 100 à 200 Angstrom, 200 à 250 Angstrom, 250 à 280 Angstrom, 280 à 300 Angstrom, 300 à 400 Angstrom, ou une longueur de 200 à 300 Angstrom.

7. Procédé selon la revendication 5, dans lequel le lieur ne comprend pas de polynucléotides.

8. Procédé selon la revendication 5, dans lequel le lieur est un PEG et le procédé comprend en outre l'étape suivante :
(vii) la transcription *in vitro* de la matrice d'affichage d'ADN de la matrice d'affichage d'ADN bicaténaire, de manière à préparer une banque comprenant des aptamères d'ARN.

9. Procédé selon la revendication 5, dans lequel le lieur est un PEG et les amorces avant sont conjuguées à des billes de telle sorte que des billes revêtues de matrice d'affichage d'ADN soient produites.

10. Procédé selon la revendication 5, dans lequel le lieur est un PEG et les amorces avant sont conjuguées à des billes, et le procédé comprend en outre (viii) la transcription *in vitro* des matrices d'affichage d'ADN sur des billes comprenant la matrice d'affichage d'ADN bicaténaire, de manière à préparer une banque comprenant des aptamères d'ARN.

11. Procédé selon l'une quelconque des revendications 5 à 10, dans lequel le conjugué NTP-lieur-ADN ou NTP-PEG-ADN est produit par un procédé comprenant les étapes suivantes :
(i) la conjugaison d'aminoallyl nucléoside triphosphate (aa-NTP) avec un N-hydroxysuccinimide (NHS)-PEG-maléimide (NHS-PEG) formant un NTP-PEG-maléimide (NTP-PEG) ;
(ii) la conjugaison d'un oligonucléotide d'ADN fonctionnalisé avec un thiol réduit (thiol-ADN) avec le maléimide sur le NTP-PEG formant un NTP-PEG-ADN ;
et
(iii) la purification du conjugué NTP-PEG-ADN.

12. Procédé selon la revendication 9 ou la revendication 10, dans lequel la PCR est une PCR en émulsion, où chaque bille affiche plusieurs copies d'une séquence de brins codants individuelle ; de préférence dans lequel l'émulsion est rompue et l'ADN bicaténaire sur les billes est dénaturé en ADN monocaténaire.

13. Procédé selon la revendication 5, dans lequel chaque brin codant d'affichage d'ADN dans la population comprend en outre un codon de début de traduction, le lieur est un PEG et les amorces avant sont conjuguées à des billes ; et dans lequel le procédé comprend en outre les étapes suivantes :
(vii) la transcription *in vitro* de la matrice d'affichage d'ADN sur des billes comprenant la matrice d'affichage d'ADN bicaténaire produisant une fusion ADN-ARN comprenant une portion ADN et une portion ARN ; et
(viii) la traduction *in vitro* de la portion ARN afin de produire une population de fusions ARN-protéine, ce qui permet de produire une banque de protéines.

14. Procédé selon la revendication 13, dans lequel la région codant l'ADN dans chaque brin codant est une région de banque aléatoire, et le procédé comprend en outre l'étape suivante :
(ix) la sélection d'une fusion ARN-protéine souhaitée sur la base de la liaison de fusion ou de l'activité, ce qui permet de sélectionner une protéine cible souhaitée et ledit acide nucléique codant ladite protéine.

15. Procédé selon la revendication 13 ou la revendication 14, dans lequel la fusion ARN-protéine comprend un ARNm lié à la protéine par le biais d'une liaison puromycine.

16. Procédé selon l'une quelconque des revendications 9 à 14, dans lequel la bille ou les billes conjuguées à une amorce avant sont formées par une liaison amino formée entre des billes fonctionnalisées par un acide carboxylique et une amorce avant fonctionnalisée par une aminé.

17. Procédé selon la revendication 14, dans lequel :
(a) la protéine souhaitée est un anticorps, un fragment d'anticorps, une aptazyme ou une enzyme ; ou
(b) la protéine souhaitée est choisie par FACS.

18. Procédé selon la revendication 14, dans lequel :
(a) le nucléotide triphosphate (aa-NTP) est l'adénosine triphosphate (ATP), le guanosine triphosphate (GTP), le cytidine triphosphate (CTP), le 5-méthyluridine triphosphate (m5UTP), l'uridine triphosphate (UTP), ou un nucléoside triphosphate non naturel ; ou
(b) le substrat ARN polymérase est une chimie de liaison alternative ajoutée pour l'attachement au PEG ; ou
(c) le PEG est le PEG3100, le PEG 3200, le PEG3300, le PEG3400, le PEG3500 ou le PEG3600.

19. Conjugué NTP-PEG-ADN comprenant un polynucléotide d'ADN et un nucléoside triphosphate (NTP), dans lequel le polynucléotide d'ADN et le NTP sont conjugués par le biais d'un PEG.
